# EUROPEAN PATENT APPLICATION

(11) **EP 4 644 466 A1**
(43) Date of publication of application: **05.11.2025**
(21) Application number: 23911775.7
(22) Date of filing: 15.12.2023
(51) Int. Cl.: C08G 77/14, A61K 8/894, A61Q 1/00, A61Q 5/00, A61Q 17/04, C08G 77/38, C08G 77/48, C08L 83/04

(54) **ORGANOPOLYSILOXANE, AND COSMETICS CONTAINING ORGANOPOLYSILOXANE**

(30) Priority: 26.12.2022 JP 2022208157
(71) Applicant: SHIN-ETSU CHEMICAL CO., LTD., Tokyo 1000005 (JP)
(72) Inventor: MORIYA, Hiroyuki, Annaka-shi, Gunma 379-0224 (JP); KONISHI, Masayuki, Tokyo 100-0005 (JP); MIYAUCHI, Masaru, Tokyo 100-0005 (JP)
(74) Representative: Mewburn Ellis LLP
(86) International application number: PCT/JP2023/045006
(87) International publication number: WO 2024/142994

(57) **Abstract**

This organopolysiloxane represented by formula (1) has excellent interfacial tension reducing ability.

R¹ₐR²_{b}R³_{c}SiO_{(4-a-b-c)/2} ( 1)

R² is a polyglycerol group represented by formula (2), and 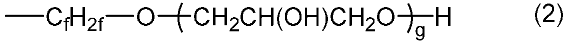 R³ is an organic group represented by formula (3).

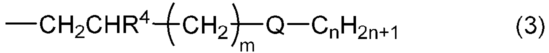

## Description

### TECHNICAL FIELD

The present invention relates to an organopolysiloxane and a cosmetic containing the same. In the present invention, compositions for use in cosmetics are referred to as cosmetics.

### BACKGROUND ART

Silicone surfactants are conventionally used widely as emulsifiers. In particular, polyglycerin-modified silicones show adhesiveness to the skin that is offered by the polyglycerin and have a light feel that is characteristic of silicones, thus finding wide use in emulsified cosmetics and the like.

Patent Document 1 proposes a method for synthesizing a polyglycerin-modified silicone as a composition by the addition reaction of a polyhydric alcohol-substituted hydrocarbon group having at least two hydroxyl groups and a silicone compound to an organohydrogenpolysiloxane.

On the other hand, it is known that specific alkyl-branched polyglycerin-modified silicones described in Patent Documents 2 and 3 are used when the formulation involves ultraviolet absorbers and oils, such as jojoba seed oil, that are poorly compatible with silicones. Unfortunately, even the use of such silicones requires large amounts of compatibilizers, such as phenyl silicones and ester oils. This fact lowers the freedom of cosmetic formulation design and makes the cosmetics feel heavy when used.

### PRIOR ART DOCUMENTS

### PATENT DOCUMENTS

Patent Document 1: JP-A 2002-179798
Patent Document 2: WO 2017/199732
Patent Document 3: JP-A 2007-269690

### SUMMARY OF INVENTION

### TECHNICAL PROBLEM

The present invention has been made in view of the circumstances discussed above. It is therefore an object of the present invention to provide an organopolysiloxane that has excellent interfacial tension reducing properties.

### SOLUTION TO PROBLEM

The present inventors carried out intensive studies directed to achieving the above object and have found that the problems described above can be solved with an organopolysiloxane represented by formula (1) below. Based on the finding, the present inventors have completed the present invention.

Thus, the present invention provides the following organopolysiloxanes and cosmetics containing the same.
1. An organopolysiloxane represented by formula (1) below:
   [Chem. 1]

   R¹ₐR²_{b}R³_{c}SiO_{(4-a-b-c)/2} (1)

   [wherein R¹ independently at each occurrence is a group selected from C₁-C₁₀ alkyl groups, phenyl group, C₇-C₁₀ aralkyl groups, and formula (1-A) and formula (1-B) below: 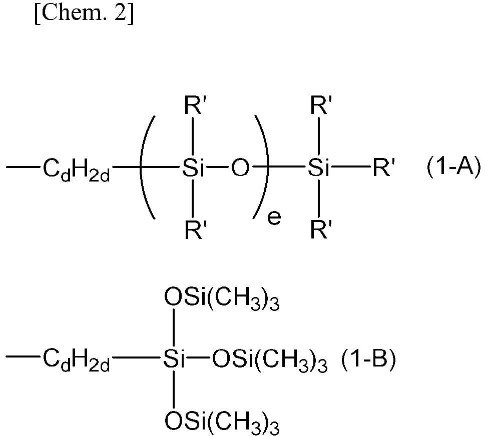 (wherein each d is a number from 2 to 6, e is 1 to 100, and R' at each occurrence is a group selected from C₁-C₁₀ alkyl groups and phenyl group);
   R² is a polyglycerol group represented by formula (2) below: 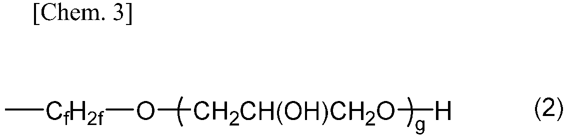 (wherein f is a number from 3 to 12 and g is a number from 1 to 10);
   R³ is an organic group represented by formula (3) below: 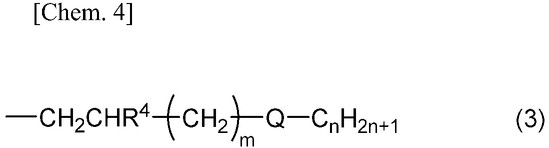 (wherein R⁴ is a hydrogen atom or a methyl group,
   Q is selected from formulas (3-1) to (3-4) below: 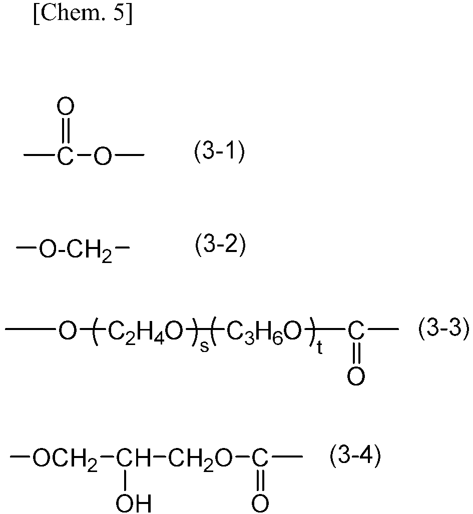
   when Q is formula (3-1), m is 8 and n is an integer of 1 to 22,
   when Q is formula (3-2), m is an integer of 1 to 10 and n is an integer of 7 to 22,
   when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
   when Q is formula (3-4), m is an integer of 1 to 10 and n is an integer of 7 to 22); and
   a, b, and c are 1.0 ≤ a ≤ 2.5, 0.001 ≤ b ≤ 1.5, 0.001 ≤ c ≤ 1.5, and 1 < a + b + c < 4].
2. The organopolysiloxane according to 1, wherein the interfacial tension between a 0.01 wt% solution of the organopolysiloxane in a light liquid paraffin and water at 25°C, as measured by a Wilhelmy method 8 hours after the formation of interface, is 25 mN/m or less.
3. A cosmetic including the organopolysiloxane described in 1 or 2.
4. The cosmetic according to 3, further including an organic ultraviolet absorber.
5. The cosmetic according to 3 or 4, further including an oil ingredient that is liquid at 25°C.
6. The cosmetic according to 5, wherein the oil ingredient that is liquid at 25°C is an ingredient selected from hydrocarbon oils, natural oils, and esters.

### ADVANTAGEOUS EFFECTS OF INVENTION

The organopolysiloxane according to the present invention performs excellently in reducing the interfacial tension. This organopolysiloxane improves oil phase properties and enables emulsification even when the formulation involves a high dose of ultraviolet absorber and a small amount of compatibilizer. Furthermore, the organopolysiloxane allows an emulsion, even when containing a large amount of natural oil, to achieve good stability and excellent feeling on use. Furthermore, a cosmetic containing the organopolysiloxane achieves excellent feeling on use.

### DESCRIPTION OF EMBODIMENTS

The present invention will be described in detail below without limiting the scope of the present invention to the following description. In the present invention, the name of an ingredient is written as the name cited in *Kesho̅hin Hyo̅ji Meisho̅* [Japanese Cosmetic Labeling Names] or in the International Nomenclature of Cosmetic Ingredients (INCI). When the names from *Kesho̅hin Hyo̅ji Meisho̅* and the INCI are the same, the INCI name or the name from *Kesho̅hin Hyo̅ji Meisho̅* is sometimes omitted.

### [Organopolysiloxanes]

An organopolysiloxane of the present invention is an organopolysiloxane represented by formula (1) below:
[Chem. 6]

R¹ₐR²_{b}R³_{c}SiO_{(4-a-b-c)/2} (1)

[wherein R¹ independently at each occurrence is a group selected from C₁-C₁₀ alkyl groups, phenyl group, C₇-C₁₀ aralkyl groups, and formula (1-A) and formula (1-B) below: 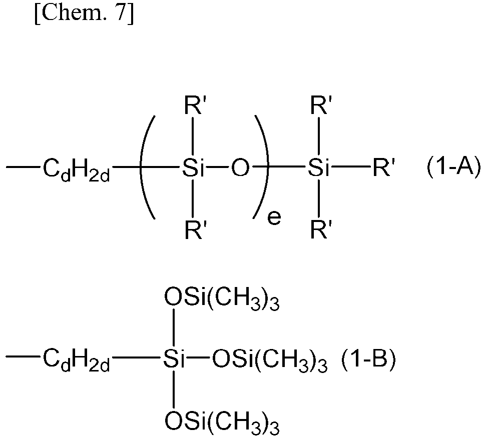 (wherein each d is a number from 2 to 6, e is 1 to 100, and R' at each occurrence is a group selected from C₁-C₁₀ alkyl groups and phenyl group);
R² is a polyglycerol group represented by formula (2) below: 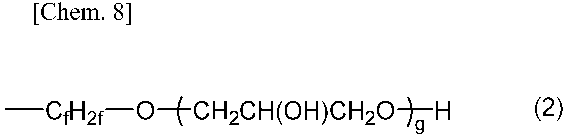 (wherein f is a number from 3 to 12 and g is a number from 1 to 10);
R³ is an organic group represented by formula (3) below: 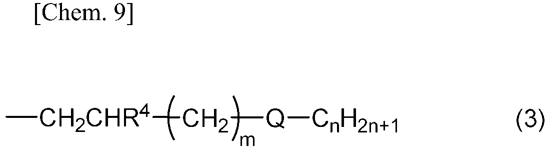 (wherein R⁴ is a hydrogen atom or a methyl group,
Q is selected from formulas (3-1) to (3-4) below: 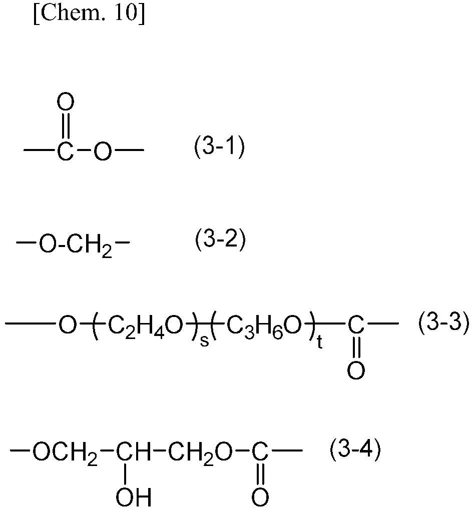
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22); and
a, b, and c are 1.0 ≤ a ≤ 2.5, 0.001 ≤ b ≤ 1.5, 0.001 ≤ c ≤ 1.5, and 1 < a + b + c < 4].

In the above average structural formula (1), R¹ independently at each occurrence is a group selected from C₁-C₁₀ alkyl groups, phenyl group, C₇-C₁₀ aralkyl groups, and formula (1-A) and formula (1-B). Examples of the C₁-C₁₀ alkyl groups include linear alkyl groups, such as methyl group, ethyl group, propyl group, butyl group, pentyl group, hexyl group, heptyl group, and octyl group, and cyclic alkyl groups, such as cyclopentyl group and cyclohexyl group.

In formulas (1-A) and (1-B), each d is a number from 2 to 6, and is preferably 2 to 4. The letter e is 1 to 100, preferably 1 to 20. R' at each occurrence is a group selected from C₁-C₁₀ alkyl groups and phenyl group, preferably a methyl group, an ethyl group, a propyl group, a butyl group, or a phenyl group, and more preferably a methyl group. R¹ is preferably a C₁-C₈ alkyl group or a phenyl group, more preferably a methyl group, an ethyl group, or a hexyl group, and still more preferably a methyl group.

R² is a polyglycerol group represented by formula (2) below: 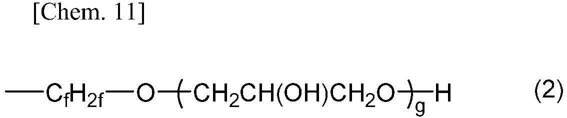 (wherein f is a number from 3 to 12 and g is a number from 1 to 10). The letter f is a number from 3 to 12, preferably 3 to 11, and more preferably 3. The letter g is a number from 1 to 10, preferably 1 to 8, and more preferably 2 to 5.

R³ is an organic group represented by formula (3) below: 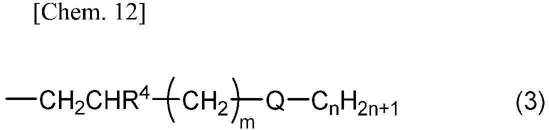 (wherein R⁴ is a hydrogen atom or a methyl group,
Q is selected from formulas (3-1) to (3-4) below: 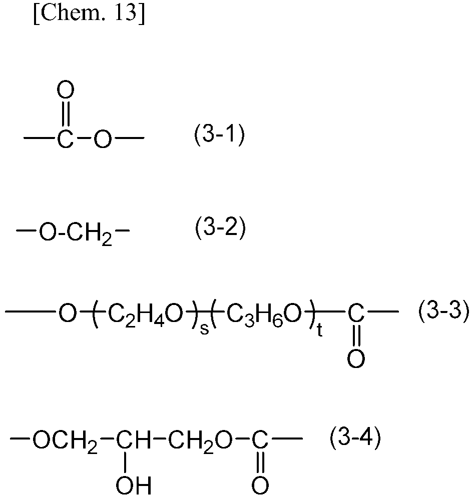
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22).

R⁴ is a hydrogen atom or a methyl group, and is preferably a hydrogen atom.

When Q is formula (3-1), m is 8 and n is an integer of 1 to 22, preferably 1 to 18.

When Q is formula (3-2), m is an integer of 1 to 10, preferably 1 to 8, and n is an integer of 7 to 22, preferably 8 to 18.

When Q is formula (3-3), m is an integer of 1 to 10, preferably 1 to 6, and n is an integer of 7 to 22, preferably 8 to 18. In formula (3-3), s is an integer of 0 to 3, preferably 0 to 2, and t is an integer of 0 to 3, preferably 0 to 2. The sum of s + t is preferably 1 or greater. The respective oxyalkylene groups are optionally bonded blockwise or randomly. Specific examples include linking groups having the following structures:

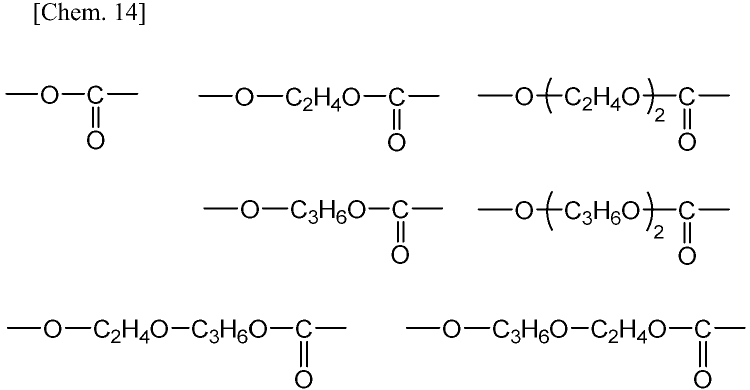

When Q is formula (3-4), m is an integer of 1 to 10, preferably 1 to 8, and n is an integer of 7 to 22, preferably 8 to 18.

The linking group Q is preferably represented by formula (3-1), (3-2), or (3-3), and more preferably represented by formula (3-1) or (3-2).

The letters a, b, and c are 1.0 ≤ a ≤ 2.5, preferably 1.2 ≤ a ≤ 2.3; 0.001 ≤ b ≤ 1.5, preferably 0.005 ≤ b ≤ 1.0; 0.001 ≤ c ≤ 1.5, preferably 0.05 ≤ c ≤ 0.5; and 1 < a + b + c < 4, preferably 1.2 < a + b + c < 2.5.

### [Production methods]

The organopolysiloxane of the present invention may be prepared by a conventional method, for example, by the addition reaction of the corresponding organohydrogenpolysiloxane with a glycerin group having a terminal carbon-carbon double bond that corresponds to formula (2) and an organic compound having a terminal carbon-carbon double bond that corresponds to formula (3) in the presence of a catalyst, such as a platinum group metal catalyst. The reaction may involve an organic solvent. Examples of the organic solvents include aliphatic alcohols, such as methanol, ethanol, 2-propanol, and butanol, aromatic hydrocarbons, such as toluene and xylene, aliphatic or alicyclic hydrocarbons, such as n-pentane, n-hexane, and cyclohexane, and halogenated hydrocarbons, such as dichloromethane, chloroform, and carbon tetrachloride. The addition reaction conditions are not particularly limited, but it is preferable to perform the reaction under reflux for 1 to 10 hours. The groups R¹ in the resultant compound may include a small amount of hydrogen atoms from the unreacted hydrosilyl groups that remain after the reaction, or a small amount of hydroxyl groups formed by the reaction. Specifically, the amount of such hydrogen atoms or hydroxyl groups is 10% or less, preferably 5% or less, of the number of all the groups R¹.

The organopolysiloxane of the present invention is preferably such that the interfacial tension between a 0.01 wt% solution of the organopolysiloxane in a light liquid paraffin and water at 25°C, as measured by a Wilhelmy method 8 hours after the formation of interface, is 25 mN/m or less. The interfacial tension is more preferably 20 mN/m or less, and still more preferably 18 mN/m or less. The interfacial tension (surface tension) is a value according to the Wilhelmy method and may be measured with a surface tensiometer. When the organopolysiloxane of the present invention is viscous, the viscosity thereof may be 8,000 to 240,000 mPa·s, and preferably 10,000 to 200,000 mPa·s. The viscosity is measured using a B-type viscometer (TVB-10, manufactured by Toki Sangyo Co., Ltd.) with a spindle No. 4, at 6 rpm and 25°C for a measurement time of 60 seconds.

In cosmetics, the organopolysiloxanes of the present invention may be used singly, or two or more may be used in combination. The content of the organopolysiloxane in the cosmetic is not particularly limited but is preferably 0.05 to 15 wt%, more preferably 0.1 to 10 wt%, and still more preferably 0.5 to 5 wt%. The following description of the present invention focuses on the use in cosmetics, but the application is not particularly limited thereto.

### [Organic ultraviolet absorbers]

The organopolysiloxane of the present invention has excellent compatibility with organic ultraviolet absorbers and allows a cosmetic containing an organic ultraviolet absorber to be favorably emulsified and to attain good feeling on use.

Any organic ultraviolet absorbers that are usually added to cosmetics may be used without limitation. Specific examples include oxybenzone-1 (INCI: Benzophenone-1), oxybenzone-2 (INCI: Benzophenone-2), oxybenzone-3 (INCI: Benzophenone-3), oxybenzone-4 (INCI: Benzophenone-4), oxybenzone-5 (INCI: Benzophenone-5), oxybenzone-6 (INCI: Benzophenone-6), oxybenzone-9 (INCI: Benzophenone-9), Homosalate (INCI), Octocrylene (INCI), t-butyl methoxydibenzoylmethane (INCI: Butyl Methoxydibenzoylmethane), Ethylhexyl Salicylate (INCI), Diethylamino Hydroxybenzoyl Hexyl Benzoate (INCI), Polysilicone-15 (INCI), octyl dimethoxybenzylidenedioxoimidazolidinepropionate (INCI: Ethylhexyl Dimethoxybenzylidene Dioxoimidazolidine Propionate), Terephthalylidene Dicamphor Sulfonic Acid (INCI), Ethylhexyltriazone (INCI), methylbis(trimethylsiloxy)silylisopentyl trimethoxycinnamate (INCI: Isopentyl Trimethoxycinnamate Trisiloxane), Drometrizole Trisiloxane (INCI), Ethylhexyl Dimethyl PABA (INCI), Isopropyl Methoxycinnamate (INCI), Ethylhexyl Methoxycinnamate (INCI), Bisethylhexyloxyphenol Methoxyphenyl Triazine (INCI), Phenylbenzimidazole Sulfonic Acid (INCI), Methylene Bisbenzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI), and Ethylhexyl Dimethoxybenzylidene Dioxoimidazolidine Propionate (INCI). Among these, Ethylhexyl Methoxycinnamate, Diethylamino Hydroxybenzoyl Hexyl Benzoate, octyl salicylate, Polysilicone-15, t-butyl methoxydibenzoylmethane, oxybenzones, Methylene Bisbenzotriazolyl Tetramethylbutylphenol, Bisethylhexyloxyphenol Methoxyphenyl Triazine, Octocrylene, Ethylhexyltriazone, and Homosalate are particularly preferable in terms of compatibility. Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

Among these, Diethylamino Hydroxybenzoyl Hexyl Benzoate, t-butyl methoxydibenzoylmethane, Methylene Bisbenzotriazolyl Tetramethylbutylphenol, Bisethylhexyloxyphenol Methoxyphenyl Triazine, and Ethylhexyltriazone are difficult to emulsify with the conventional polyglycerin-modified silicones. The organosiloxanes of the present invention exhibit good performance in emulsifying these powdered organic ultraviolet absorbers.

When the organic ultraviolet absorber is added, the content thereof is not particularly limited but is preferably 0.5 to 30 wt%, more preferably 3 to 25 wt%, and still more preferably 5 to 20 wt% of the cosmetic. If the content is more than 30 wt%, the cosmetic may feel heavy or may be highly irritating. Because the organopolysiloxane of the present invention has excellent compatibility with organic ultraviolet absorbers, the present invention produces greater effects when the content is 0.5 wt% or more.

### [Oil ingredients that are liquid at 25°C]

A single or a combination of two or more oil ingredients that are liquid at 25°C may be used. The oil ingredient that is liquid at 25°C preferably has a kinematic viscosity of 1 to 100 mm²/s, more preferably 1 to 30 mm²/s, as measured at 25°C with a Cannon-Fenske viscometer by the method described in JIS Z 8803: 2011. From the point of view of the compatibility with the organopolysiloxane of the present invention, one having a kinematic viscosity of 1 to 20 mm²/s is still more preferable. Examples of the oil ingredients that are liquid at 25°C include hydrocarbon oils, higher fatty acids, natural oils, esters, silicone oils, and fluorinated oils. In particular, hydrocarbon oils, natural oils, esters, and silicone oils are preferable from the point of view of the compatibility with organic ultraviolet absorbers, and hydrocarbon oils, natural oils, and esters are still more preferable from the point of view of the compatibility with the organopolysiloxane of the present invention.

### • Hydrocarbon oils

Examples of the hydrocarbon oils include linear or branched hydrocarbon oils. The hydrocarbon oils may be volatile or nonvolatile. Specific examples include Olefin Oligomers (INCI), Isododecane (INCI), Dodecane (INCI), Isohexadecane (INCI), Undecane (INCI), Squalane (INCI), Squalene (INCI), Mineral Oil (INCI), liquid isoparaffin, polyisobutylene, Hydrogenated Polyisobutene (INCI), and C13-15 Alkane (INCI). Among these, Dodecane, C13-15 Alkane, and Undecane are particularly preferable from the point of view of the compatibility with organic ultraviolet absorbers.

### • Higher fatty acids

Examples of the higher fatty acids include Oleic Acid (INCI), Linoleic Acid (INCI), Linolenic Acid (INCI), Arachidonic Acid (INCI), Eicosapentaenoic Acid (INCI) (EPA), Docosahexaenoic Acid (INCI) (DHA), Isostearic Acid (INCI), and Hydroxystearic Acid (INCI).

### • Natural oils

Examples of the natural oils include natural animal and vegetable oils and fats, and semi-synthetic oils and fats. Specific examples include avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), olive oil (INCI: Olea Europaea (Olive) Fruit Oil), Torreya californica oil (INCI: Torreya Californica (California Nutmeg) Oil), citronella grass oil (INCI: Cymbopogon Nardus (Citronella) Oil), Shark Liver Oil (INCI), Cod Liver Oil (INCI), Fish Liver Oil (INCI), apricot kernel oil (INCI: Kyounin Yu), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sasanqua oil (INCI: Camellia Kissi Seed Oil), Turtle Oil (INCI), camellia oil (INCI: Camellia Japonica Seed Oil), evening primrose oil (INCI: Oenothera Biennis (Evening Primrose) Oil), corn germ oil (INCI: Zea Mays (Corn) Germ Oil), rapeseed oil (INCI: Rape Shushi Yu), wheat germ oil (INCI: Triticum Vulgare (Wheat) Germ Oil), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), hydrogenated castor oil, sunflower seed oil (INCI: Helianthus Annuus (Sunflower) Seed Oil), grape seed oil (INCI: Vitis Vinifera (Grape) Seed Oil), jojoba seed oil (INCI: Simmondsia Chinensis (Jojoba) Seed Oil), macadamia nut oil (INCI: Macadamia Ternifolia Seed Oil), Mink Oil (INCI), meadowfoam oil (INCI: Limnanthes Alba (Meadowfoam) Seed Oil), cotton seed oil (INCI: Gossypium Herbaceum (Cotton) Seed Oil), coconut oil (INCI: Cocos Nucifera (Coconut) Oil), Hydrogenated Coconut Oil (INCI), egg yolk oil (INCI: Egg Oil), and Argania Spinosa Kernel Oil (INCI). Among these, jojoba seed oil, Argania Spinosa Kernel Oil, meadowfoam oil, macadamia nut oil, olive oil, sunflower seed oil, and sesame oil are difficult to emulsify with the conventional polyglycerin-modified silicones. The organosiloxanes of the present invention exhibit good performance in emulsifying these oils.

### • Esters

The esters are liquid oils that are condensation products of a C₁-C₂₀ fatty acid and a C₁-C₂₀ alcohol and, similarly to the natural oils, may have an animal-derived or vegetable-derived composition. Examples include monoesters and polyesters, such as diesters and triesters. Specific examples include Diisobutyl Adipate (INCI), dihexyldecyl adipate, Diheptylundecyl Adipate (INCI), n-alkyl glycol monoisostearates, such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Triethylhexanoin (INCI), Trimethylolpropane Triethylhexanoate (INCI), Pentaerythrityl Tetraethylhexanoate (INCI), cetyl octanoate (INCI: Cetyl Ethylhexanoate), octyldodecyl esters, such as octyldodecyl stearoyloxystearate (INCI: Octyldodecyl Stearoyl Stearate), Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), cocoalkyl (caprylate/caprate) (INCI: Coco-Caprylate/Caprate), glyceryl tri(caprylate/caprate) (INCI: Caprylic/Capric Triglyceride), palmitic acid esters, such as Isopropyl Palmitate (INCI), ethylhexyl palmitate (INCI: Ethylhexyl Isopalmitate), and hexyldecyl palmitate (INCI: Isocetyl Palmitate, Hexyldecyl Palmitate), Cholesteryl Hydroxystearate (INCI), myristic acid esters, such as Isopropyl Myristate (INCI), Octyldodecyl Myristate (INCI), and Myristyl Myristate (INCI), Ethylhexyl Laurate (INCI), Hexyl Laurate (INCI), Dioctyldodecyl Lauroyl Glutamate (INCI), Isopropyl Lauroyl Sarcosinate (INCI), Diisostearyl Malate (INCI), and glyceride oils, such as Glyceryl Acetate (INCI) and Glyceryl Stearate (INCI). Among these, Cetyl Ethylhexanoate, Triethylhexanoin, Neopentyl Glycol Diethylhexanoate, Octyldodecyl Myristate, Ethylhexyl Palmitate, Hexyl Laurate, cocoalkyl (caprylate/caprate), and glyceryl tri(caprylate/caprate) are particularly preferable from the point of view of the compatibility with the organic ultraviolet absorbers.

### • Silicone oils

Examples of the silicone oils include linear or branched dimethicones having a low to high viscosity, such as Trisiloxane (INCI), volatile dimethicone (INCI: Dimethicone), low-viscosity dimethicone (INCI: Dimethicone), Cyclotetrasiloxane (INCI), Cyclopentasiloxane (INCI), Cyclohexasiloxane (INCI), Methyl Trimethicone (INCI), Caprylyl Methicone (INCI), Phenyl Trimethicone (INCI), methylphenylpolysiloxane (INCI: Diphenyl Dimethicone), Diphenylsiloxy Phenyl Trimethicone (INCI), Ethyl Methicone (INCI), Ethyl Trisiloxane (INCI), and Hydrogen Dimethicone (INCI); Amodimethicone (INCI), and Aminopropyl Dimethicone (INCI). Among these, Methyl Trimethicone, Trisiloxane, volatile dimethicone, low-viscosity dimethicone, and Diphenylsiloxy Phenyl Trimethicone are particularly preferable from the point of view of the compatibility with the organic ultraviolet absorbers. Examples of the commercially available silicone oils include KF-96L-1cs, KF-96L-1.5cs, KF-96L-2cs, KF-96A-6cs, KF-4422, KF-4418, KF-56A, and KF-995 manufactured by Shin-Etsu Chemical Co., Ltd. Examples further include silicone rubbers, such as high degree of polymerization gum-like dimethicone (INCI: Dimethicone), gum-like amodimethicone (INCI: Amodimethicone), and gum-like dimethylsiloxane-methylphenylsiloxane copolymer; cyclic organopolysiloxane solutions of silicone gums and rubbers, amino acid-modified silicones, fluorine-modified silicones, silicone resins, and silicone resin solutions. Examples of the commercially available silicone oils include KF-54 and KF-54HV. The organopolysiloxane of the present invention can be used to control the compatibility with these oils so as to prepare cosmetics utilizing phase separation technology.

### • Fluorinated oils

Examples of the fluorinated oils include perfluoropolyethers, such as polyperfluoromethyl isopropyl ether (INCI: Polyperfluoromethylisopropyl Ether), and perfluorocarbons, such as Perfluorodecalin (INCI) and Perfluorohexane (INCI).

When the oil that is liquid at 25°C is added, the content thereof is not particularly limited but is preferably 1 to 80 wt%, more preferably 2 to 50 wt%, and still more preferably 5 to 40 wt% of the cosmetic.

### [Other ingredients]

The cosmetic of the present invention may contain various other ingredients that are usually added to cosmetics. For example, such additional ingredients are (1) oils other than the oil ingredients that are liquid at 25°C, (2) aqueous ingredients, (3) surfactants other than the organopolysiloxanes of the present invention, (4) powders, (5) compositions including a crosslinked organopolysiloxane and an oil that is liquid at room temperature, (6) film-forming agents, (7) antiperspirants, (8) antimicrobial agents, and (9) other additives. These may be used singly, or two or more may be used in appropriate combination. These ingredients are appropriately selected and used in accordance with, for example, the type of cosmetic, and the content thereof may be a known content in accordance with, for example, the type of cosmetic. When any of the ingredients described hereinabove also appears as an optional ingredient, the description given hereinabove takes precedence.

### (1) Oils other than the oils that are liquid at 25°C

The oils other than the oils that are liquid at 25°C may be solid or semi-solid at 25°C. Examples of such oils that may be used include natural animal and vegetable oils and fats, semi-synthetic oils and fats, hydrocarbon oils, higher fatty acids, higher alcohols, esters, silicones, and fluorinated oils.

The oil ingredients that are solid at 25°C preferably have a melting point of 40°C or above, more preferably 60 to 110°C. Any such ingredients that are usually added to cosmetics may be used without limitation, with examples including waxes, hydrocarbons, esters, higher alcohols, and higher fatty acids.

Specific examples include vegetable waxes, such as carnauba wax, sugarcane wax, candelilla wax, refined candelilla wax, rice wax, wood wax, jojoba wax, kapok wax, rice bran wax, bayberry fruit wax, shea butter, cacao butter, Rhus Succedanea Fruit Wax (INCI), Montan Wax (INCI), and hydrogenated castor oil isostearate; animal waxes, such as beeswax, beef tallow, beef bone fat, Lard (INCI), Horse Fat (INCI), mutton tallow, Lanolin (INCI), Ericerus pela wax, shellac wax, and spermaceti; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffins, polyethylene, ceresin, ozokerite, and microcrystalline wax; higher alcohols, such as stearyl alcohol, behenyl alcohol, and cetanol; wax esters, such as synthetic beeswax; amino acid stearyl alcohols, such as dioctyldodecyl lauroyl glutamate, dioctyldodecyl lauroyl glutamate, and dioctyldodecyl lauroyl glutamate; fatty acids, such as stearic acid and behenic acid; silicone waxes, such as acrylic silicone resins of acrylic-silicone graft or block copolymers (for example, acrylic-silicone graft copolymer: KP-561P, manufactured by Shin-Etsu Chemical Co., Ltd.); and derivatives thereof. One, or two or more selected from the above are preferably used.

### (2) Aqueous ingredients

Any aqueous ingredients that are usually added to cosmetics may be used without limitation. Specific examples of the aqueous ingredients include water, moisturizers, and water-soluble polymer compounds. These may be used singly, or two or more may be used in appropriate combination.

Examples of water include purified water commonly used in cosmetics, distilled water from fruits and plants, and seawater, hot spring water, and peat water defined in *Kesho̅hin Hyo̅ji Meisho̅.* The amount in which water is added is preferably 10 to 90 wt%, more preferably 30 to 85 wt%, and still more preferably 50 to 80 wt% of the whole of the cosmetic.

Examples of the moisturizers include lower alcohols, preferably those having 2 to 5 carbon atoms, such as ethanol (INCI: Alcohol) and isopropanol (INCI: Isopropyl Alcohol); sugar alcohols, such as Sorbitol (INCI), Maltose (INCI), and Xylitol (INCI); polyhydric alcohols, such as BG (INCI: Butylene Glycol), PG (INCI: Propylene Glycol), DPG (INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; Glucose (INCI), Glyceryl Glucoside (INCI), Betaine (INCI), Sodium Chondroitin Sulfate (INCI), Sodium PCA (INCI), Methyl Gluceth-10 (INCI), and Methyl Gluceth-20 (INCI). Examples further include sterols, such as Cholesterol (INCI), sitosterol (INCI: Beta-Sitosterol), Phytosterols (INCI), and Lanosterol (INCI). Examples of the moisturizers further include hyaluronic acid, egg yolk lecithin, soybean lecithin, phosphatidylcholine, phosphatidylethanolamine, phosphatidylserine, phosphatidylglycerol, phosphatidylinositol, and sphingophospholipid. The amount in which the moisturizer is added is preferably 1 to 90 wt%, more preferably 3 to 50 wt%, and still more preferably 5 to 20 wt% of the whole of the cosmetic.

Examples of the water-soluble polymer compounds include natural water-soluble polymer compounds, such as carrageenan, hyaluronates, and xanthan gum; semi-synthetic water-soluble polymer compounds, such as hydroxyethyl cellulose, hydroxypropylmethyl cellulose, and carboxymethyl cellulose; synthetic water-soluble polymer compounds, such as polyvinyl alcohol, polyvinylpyrrolidone, and carboxyvinyl polymer; and inorganic water-soluble polymer compounds, such as bentonite and laponite. The amount in which the water-soluble polymer compound is added is preferably 0.01 to 1 wt% of the whole of the cosmetic.

### (3) Surfactants other than the organopolysiloxanes of the present invention

The surfactants other than the organopolysiloxanes of the present invention may be nonionic, anionic, cationic, or amphoteric and any surfactants usually added to cosmetics may be used without limitation. In particular, preferred surfactants are partially crosslinked polyether-modified silicones, partially crosslinked polyglycerin-modified silicones, linear or branched polyoxyethylene-modified organopolysiloxanes, linear or branched polyoxyethylene polyoxypropylene-modified organopolysiloxanes, linear or branched polyoxyethylene/alkyl co-modified organopolysiloxanes, linear or branched polyoxyethylene polyoxypropylene/alkyl co-modified organopolysiloxanes, linear or branched polyglycerin-modified organopolysiloxanes, and linear or branched polyglycerin/alkyl co-modified organopolysiloxanes.

In these surfactants, the content of hydrophilic polyoxyethylene groups, polyoxyethylene polyoxypropylene groups, or polyglycerin residues is preferably 10 to 70 wt% of the molecule.

Examples of the partially crosslinked polyether-modified silicones and the partially crosslinked polyglycerin-modified silicones include (Dimethicone/(PEG-10/15) Crosspolymer (INCI), (PEG-15/Lauryl Dimethicone) Crosspolymer (INCI) , (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the partially crosslinked polyether-modified silicone or the partially crosslinked polyglycerin-modified silicone is used, the crosslinked organopolysiloxane preferably forms a composition with an oil that is liquid at room temperature in such a manner that the crosslinked organopolysiloxane is swollen with its own weight or more of the liquid oil.

The liquid oil may be a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Examples of the commercial crosslinked organopolysiloxanes swollen with liquid oils include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd.

Exemplary surfactants that are not crosslinked organopolysiloxanes include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), and Lauryl Polyglyceryl-3 Polydimethylsiloxyethyl Dimethicone (INCI).

Exemplary commercial products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

For any types of surfactants, the amount is preferably 0.1 to 20 wt% of the whole of the cosmetic. When the amount is 0.1 wt% or more, the surfactant can fully fulfill its dispersing and emulsifying functions. When the amount is 20 wt% or less, the cosmetic will not give a sticky feeling on use and is therefore preferable. The HLB of the surfactant is not limited but is preferably 2 to 14.5 in order to maintain the water resistance of the cosmetic.

### (4) Powders

Examples of the powders include coloring pigments, inorganic powders, metal powders, organic powders, and inorganic/organic composite powders. The powders will be specifically described below.

### • Coloring pigments

The coloring pigments are not particularly limited and any pigments that are usually added to give colors to cosmetics may be used. Examples include red iron oxide (INCI: Iron Oxides), yellow iron oxide (INCI: Iron Oxides), white titanium oxide (INCI: Titanium Dioxide), black iron oxide (INCI: Iron Oxides), Ultramarines (INCI), iron blue (INCI: Ferric Ferrocyanide, Ferric Ammonium Ferrocyanide), Manganese Violet (INCI), Cobalt Titanium Oxide (INCI), chromium hydroxide (INCI: Chromium Hydroxide Green), chromium oxide (INCI: Chromium Oxide Greens), Cobalt Aluminum Oxide (INCI), fired (titanium/titanium oxide) (INCI: Titanium/Titanium Dioxide), Lithium Cobalt Titanate (INCI), sintered (iron oxide/titanium oxide), composites doped with dissimilar metals, such as iron oxide doped titanium oxide (INCI: Iron Oxides, Titanium Dioxide), Titanium Nitride (INCI), ferrous hydroxide (INCI: Iron Hydroxide), inorganic brown pigments, such as γ-iron oxide, inorganic yellow pigments, such as loess, and colored pigments, such as lakes of tar dyes and lakes of natural dyes.

The pigments may have any shapes, such as spherical, approximately spherical, rod-like, spindle-like, petal-like, strip-like, and amorphous shapes. The geometric forms of the pigments are not particularly limited as long as the pigments can impart colors to the cosmetics.

### • Inorganic powders

Examples of the inorganic powders include fine particles of zirconium oxide (INCI: Zirconium Dioxide), Zinc Oxide (INCI), Cerium Oxide (INCI), Magnesium Oxide (INCI), Barium Sulfate (INCI), Calcium Sulfate (INCI), Magnesium Sulfate (INCI), Calcium Carbonate (INCI), Magnesium Carbonate (INCI), Talc (INCI), Mica (INCI), Kaolin (INCI), Synthetic Fluorphlogopite (INCI), synthetic ferrous phlogopite, black mica (INCI: Biotite), Potassium Silicate (INCI), Silica (INCI), Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI), Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), Zeolite (INCI), Alumina (INCI), Aluminum Hydroxide (INCI), Boron Nitride (INCI), and Glass (INCI).

Inorganic coloring pearl pigments include pearl agents, such as Mica (INCI) coated with titanium oxide (INCI: Titanium Dioxide) and Synthetic Fluorophlogopite (INCI) coated with titanium oxide (INCI: Titanium Dioxide), and pearl pigments, such as Bismuth Oxychloride (INCI), Bismuth Oxychloride (INCI) coated with titanium oxide (INCI: Titanium Dioxide), Talc (INCI) coated with titanium oxide (INCI: Titanium Dioxide), fish scale flakes, and coloring mica coated with titanium oxide (INCI: Titanium Dioxide). These pigments may be surface-treated by a known method generally used for cosmetics or may be free from such surface treatment.

### • Metal powders

Examples of the metal powders include metal fine particles of aluminum (INCI: Aluminum, Aluminum Powder), copper (INCI: Copper Powder), silver (INCI: Silver Powder), and Gold (INCI).

### • Organic powders

Examples of the organic powders include powders of silicone, polyamide, polyacrylic acid/acrylic acid ester, polyester, Polyethylene (INCI), Polypropylene (INCI), Polystyrene (INCI), styrene/acrylic acid copolymer, divinylbenzene/styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon, phenolic resin, epoxy resin, and polycarbonate.

In particular, examples of the silicones include silicone resin particles; Polymethylsilsesquioxane (INCI), silicone rubber powder, silicone resin-coated silicone rubber powder; Vinyl Dimethicone/Methicone Silsesquioxane Crosspolymer (INCI), Diphenyl Dimethicone/Vinyl Diphenyl Dimethicone/Silsesquioxane Crosspolymer (INCI), Polysilicone-1 Crosspolymer (INCI), and Polysilicone-22 (INCI).

Examples of the commercial silicone powders include KMP-590, KMP-591, KMP-592, KMP-597, KMP-598, KSP-100, KSP-101, KSP-102, KSP-105, KSP-300, KSP-411, KSP-441, KM-9729, KM-440, and KSP-100W manufactured by Shin-Etsu Chemical Co., Ltd.

Examples further include metal soaps. Specific examples include powders of Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), and Potassium Cetyl Phosphate (INCI).

Examples further include organic dyes. Specific examples include tar dyes, such as red 3, red 104 (1) (INCI: Red 28, Red 28 Lake), red 106, red 201 (INCI: Red 6), red 202 (INCI: Red 7), red 204, red 205, red 220 (INCI: Red 34), red 226 (INCI: Red 30), red 227 (INCI: Red 33, RED 33 Lake), red 228 (INCI: Red 36), red 230 (1) (INCI: Red 22, Red 22 Lake), red 230 (2), red 401, red 505, yellow 4 (INCI: Yellow 5), yellow 5 (INCI: Yellow 6, Yellow 6 Lake), yellow 202 (1) (INCI: Yellow 8), yellow 203 (INCI: Yellow 10, Yellow 10 Lake), yellow 204 (INCI: Yellow 11), yellow 401, blue 1 (INCI: Blue 1, Blue 1 Lake), blue 2, blue 201, blue 205 (INCI: Blue 4), blue 404, green 3 (INCI: Green 3, Green 3 Lake), green 201 (INCI: Green 5), green 202 (INCI: Green 6), green 204 (INCI: Green 8), green 205, orange 201 (INCI: Orange 5), orange 203 (INCI: Pigment Orange 5), orange 204, orange 205 (INCI: Orange 4, Orange 4 Lake), orange 206 (INCI: Orange 10), and orange 207 (INCI: Orange 11), and natural dyes, such as Cochineal (INCI), Laccaic Acid (INCI), safflower red (INCI: Carthamus Tinctorius (Safflower) Flower Extract), Lithospermum Officinale Root Extract (INCI), gardenia yellow, and gardenia blue (INCI: Hydrolyzed Gardenia Florida Extract).

### • Inorganic/organic composite powders

Examples of the inorganic/organic composite powders include composite powders in which the surface of an inorganic powder is coated with an organic powder by a publicly known method.

The powders described hereinabove may be surface-treated. From the point of view of the water resistance of the cosmetic, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyls and phosphate salts, surfactants, amino acids, such as N-acylglutamic acid, and metal soaps, such as aluminum stearate and magnesium myristate.

Silicone treatment agents are more preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and Acrylates/Tridecyl Acrylate/Triethoxysilylpropyl Methacrylate/Dimethicone Methacrylate Copolymer (INCI). Specific examples of the silicone treatment agents include AES-3083, KF-99P, KF-9901, KF-9908, KF-9909, KP-574, and KP-541 manufactured by Shin-Etsu Chemical Co., Ltd. The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions including a crosslinked organopolysiloxane and an oil that is liquid at 25°C

In the composition including a crosslinked organopolysiloxane and an oil that is liquid at 25°C, the crosslinked organopolysiloxane is preferably swollen by containing its own weight or more of the liquid oil. The liquid oil may be a silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Unlike the ingredients (3), the ingredients (5) are compounds that do not have a polyether or polyglycerin structure in the molecular structure. Specific examples include (Dimethicone/Vinyl Dimethicone) Crosspolymer (INCI), (Dimethicone/Phenyl Vinyl Dimethicone) Crosspolymer (INCI), (Vinyl Dimethicone/Lauryl Dimethicone) Crosspolymer (INCI), and (Lauryl Polydimethylsiloxyethyl Dimethicone/Bisvinyl Dimethicone) Crosspolymer (INCI).

Examples of the commercial compositions including a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-forming agents

The film-forming agent is added mainly for the purpose of further increasing the durability of the effects of the cosmetic. The film-forming agents are not particularly limited but are preferably silicone compositions from the point of view of imparting water repellency. Specifically, for example, trimethylsiloxysilicate, acrylic-silicone coating agents, silicone-modified norbornenes, and silicone-modified pullulans may be used.

Examples of the silicone compositions as the film-forming agents include Trimethylsiloxysilicate (INCI), (Acrylates/Dimethicone) Copolymer (INCI), (Norbornene/Tris(trimethylsiloxy)silylnorbornene) Copolymer (INCI), and pullulan tri(trimethylsiloxy)silylpropylcarbamate (INCI: Trimethylsiloxysilylcarbamoyl Pullulan).

The film-forming agent may be added to the cosmetic after being previously dissolved into an oil that is liquid at room temperature. The liquid oil may be a silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), and Butyl Acetate (INCI).

Specific examples of the commercial silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) Antiperspirants

When the cosmetic according to the present invention is a deodorant, an antiperspirant may be optionally added. The antiperspirant is not particularly limited as long as it shrinks the skin pores to suppress perspiration. General such ingredients may be widely used. Examples include halogenated hydroxyaluminums, such as chlorohydroxyaluminum and allantoin chlorohydroxyaluminum, aluminum halides, such as aluminum chloride, allantoin aluminum salt, tannic acid, persimmon tannin, aluminum potassium sulfate, zinc oxide, zinc p-phenolsulfonate, burnt alum, tetrachloro(Al/zirconium) hydrate, and trichlorohydrex glycine (Al/zirconium). In order to obtain high effects, in particular, the antiperspirant active ingredient is preferably selected from the group consisting of aluminum halides, halogenated hydroxyaluminums, and complexes or mixtures thereof with zirconyl oxyhalides and zirconyl hydroxyhalides.

The antiperspirant may be used as a solution in water, or a powder thereof may be added directly to the preparation. Commercially available antiperspirants may be used. The commercially available product may be in the form of a mixed ingredient containing other ingredients. The content of the antiperspirant is not particularly limited and may be changed appropriately depending on the amounts of other ingredients that are blended. In order to obtain a deodorant with excellent antiperspirant effects and to reduce the skin irritation of the deodorant, the content is preferably in the range of 0.001 to 30 wt%, and more preferably in the range of 0.01 to 20 wt% of the whole of the deodorant.

### (8) Antimicrobial agents

The antimicrobial agents are not particularly limited as long as the ingredients can provide deodorizing effects by suppressing the proliferation of bacteria resident on the skin that produce odor-causing substances. For example, such antimicrobial agents as triclosan, benzalkonium chloride, benzethonium chloride, chlorhexidine hydrochloride, chlorhexidine gluconate, halocarban, and isomethylphenol are generally used. Furthermore, for example, crude drug-derived essential oils or extracts having antibacterial properties, such as green tea dry distillation extract, may be added. Examples of the antimicrobial agents that are crude drug-derived essential oils or extracts having deodorizing effects include green tea extract, lavender extract, Scutellaria baicalensis Georgi extract, Coptis japonica extract, Phellodendron Amurense Bark extract, Artemisia capillaris extract, Aloe arborescens extract, Sophora flavescens root extract, Sasa veitchii leaf extract, Allium sativum extract, Hamamelis extract, black tea extract, Salvia officinalis leaf extract, Zanthoxylum fruit extract, Zingiber Officinale root extract, Acorus Calamus root extract, Hedera Helix extract, Houttuynia Cordata extract, Prunus Persica fruit extract, Prunus Persica leaf extract, Mentha Piperita leaf extract, Cnidium Officinale root extract, Eucalyptus Globulus leaf extract, Arachis Hypogaea seedcoat extract, Ganoderma Lucidum extract, and Sanguisorba Officinalis root extract. The plant extracts may be used singly, or two or more may be used in combination.

### (9) Other additives

Other additives include oil-soluble gelling agents, ultraviolet absorbing and scattering agents, preservatives, fragrances, salts, antioxidants, pH adjusters, chelating agents, algefacients, anti-inflammatory agents, skin beautifying ingredients (such as whitening agents, cell activators, skin roughness improvers, circulation promoting ingredients, skin astringents, and antiseborrheic agents), vitamins, amino acids, nucleic acids, hormones, and clathrate compounds.

### • Oil-soluble gelling agents

Examples of the oil-soluble gelling agents include metal soaps, such as aluminum stearate, magnesium stearate, and zinc myristate; amino acid derivatives, such as Lauroyl Glutamic Acid (INCI) and α,γ-di-n-butylamine; dextrin fatty acid esters, such as Dextrin Palmitate (INCI), Dextrin Isostearate (INCI), Dextrin Myristate (INCI), inulin stearate (INCI: Stearoyl Inulin), and Dextrin Palmitate/Ethylhexanoate (INCI); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organically modified clay minerals of hectorite, such as Disteardimonium Hectorite (INCI) and Stearalkonium Hectorite (INCI); and Stearalkonium Bentonite (INCI).

### • Ultraviolet absorbing and scattering agents

Examples of the ultraviolet absorbing and scattering agents include particles that absorb and scatter ultraviolet rays, such as titanium oxide fine particles, iron-containing titanium oxide fine particles, zinc oxide fine particles, cerium oxide fine particles, and composites thereof. These ultraviolet absorbing and scattering particles may be dispersed in an oil beforehand.

The oil may be a silicone oil, a hydrocarbon oil, an ester oil, a natural oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI). Specific examples of the oil dispersions of the ultraviolet absorbing and scattering particles include SPD series (product name), in particular, SPD-T5, SPD-Z5, SPD-T5L, SPD-T7, and SPD-Z7L manufactured by Shin-Etsu Chemical Co., Ltd.

### • Preservatives

Examples of the preservatives include p-oxybenzoic acid alkyl esters, benzoic acid, sodium benzoate, sorbic acid, potassium sorbate, and phenoxyethanol. Examples of the antimicrobial agents include benzoic acid, salicylic acid, carbolic acid, sorbic acid, p-oxybenzoic acid alkyl esters, p-chloro-m-cresol, hexachlorophene, trichlorocarbanilide, photosensitizers, and phenoxyethanol.

### • Fragrances

The fragrances include natural fragrances and synthetic fragrances. Examples of the natural fragrances include plant fragrances separated from, for example, flowers, leaves, wood, and pericarps; and animal fragrances, such as musk and civet. Examples of the synthetic fragrances include hydrocarbons, such as monoterpenes; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

The salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include salts of amines, such as triethanolamine, and salts of amino acids, such as glutamic acid. Furthermore, use may be made of, for example, salts of hyaluronic acid and chondroitin sulfuric acid, aluminum zirconium glycine complex, and acid-alkali neutral salts used in cosmetic formulations.

### • Antioxidants

The antioxidants are not particularly limited. Examples include carotenoids, ascorbic acid and salts thereof, ascorbyl stearate, tocophenol, tocophenol acetate, tocopherol, p-t-butylphenol, butylhydroxyanisole, dibutylhydroxytoluene, phytic acid, ferulic acid, thiotaurine, hypotaurine, sulfite salts, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • pH adjusters

Examples of the pH adjusters include lactic acid, citric acid, glycolic acid, succinic acid, tartaric acid, dl-malic acid, potassium carbonate, sodium hydrogen carbonate, and ammonium hydrogen carbonate.

### • Chelating agents

Examples of the chelating agents include alanine, sodium edetate, sodium polyphosphate, sodium metaphosphate, and phosphoric acid.

### • Algefacients

Examples of the algefacients include L-menthol and camphor.

### • Anti-inflammatory agents

Examples of the anti-inflammatory agents include allantoin, glycyrrhizic acid and salts thereof, glycyrrhetinic acid and stearyl glycyrrhetinate, tranexamic acid, and azulene.

### • Skin beautifying ingredients

Examples of the skin beautifying ingredients include whitening agents, such as placenta extract, arbutin, glutathione, and Saxifraga Sarmentosa extract; cell activators, such as royal jelly, photosensitizers, cholesterol derivatives, and calf blood extract; skin roughness improvers; circulation promoting ingredients, such as nonylic acid vanillylamide, benzyl nicotinate, β-butoxyethyl nicotinate, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamins A, such as vitamin A oils, retinol, retinyl acetate, and retinyl palmitate; vitamins B, including vitamins B₂, such as riboflavin, riboflavin butyrate, and flavin adenine nucleotide, vitamins B₆, such as pyridoxine hydrochloride, pyridoxine dioctanoate, and pyridoxine tripalmitate, vitamin B₁₂ and derivatives thereof, and vitamin B15 and derivatives thereof; vitamins C, such as L-ascorbic acid, L-ascorbyl dipalmitate, sodium L-ascorbyl-2-sulfate, and dipotassium L-ascorbyl diphosphate; vitamins D, such as ergocalciferol and cholecalciferol; vitamins E, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinamide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

The cosmetic of the present invention may be applied to various products, such as, but not particularly limited to, lotions, beauty essences, milky lotions, creams, hair care products, foundations, makeup bases, sunscreens, concealers, blush colors, lipsticks, glosses, balms, mascaras, eye shadows, eyeliners, body makeups, deodorants, and nail cosmetics. Among these, in particular, makeup cosmetics and preparations with sunscreen effects are preferable, with examples including foundations, makeup bases, sunscreens, concealers, blush colors, lipsticks, and glosses. The type of formulation of the cosmetic of the present invention may be selected from various types of formulations, such as liquids, creams, solids, pastes, gels, mousses, souffles, clays, powders, and sticks.

The cosmetics described above may be in the form of emulsion, oil-based, or water-based. When an oily feel is desired, an oil-based composition or an emulsion is selected. The emulsion may be in any form of O/W emulsion, W/O emulsion, O/W/O emulsion, or W/O/W emulsion. When a fresh feel is desired, a water-based composition or a powder composition may be selected. In any of these cases, good-quality cosmetics may be obtained. In the present invention, the term "water-based composition" refers to a composition that does not purposefully contain an oil. The term "oil-based composition" refers to a composition that does not purposefully contain an aqueous ingredient. The term "powder composition" refers to a composition in which fine particles or the like are coated with an aqueous phase or an aqueous phase is coated with a powder and which has a powder appearance but, immediately after applied to the skin, releases the aqueous phase to produce a dewy feel. Among these compositions, an emulsion composition using the organopolysiloxane of the present invention as an emulsifier is particularly preferable.

### EXAMPLES

Hereinafter, the present invention will be described in detail by presenting Production Examples, Comparative Production Examples, and Examples and Comparative Examples of cosmetics. However, the present invention is not limited to the following Examples. Unless otherwise specified, "%" in the compositions below means "wt%" and indicates the wt% of an ingredient relative to the weight of the total of each example taken as 100 wt%. The amount indicates the amount of the product described. In the formulas below, C₆H₅- denotes a phenyl group. The interfacial tension (surface tension) is a value measured by the Wilhelmy method at 25°C with a surface tensiometer (device name: DY-700, manufactured by Kyowa Interface Science Co., Ltd.) 8 hours after the formation of interface. In the formulas, terminals that are not bonded and are not specifically described are "-CH₃", and the terminal of a double bond is "CH₂=CH₂-".

### [Production Example 1]

A reactor was charged with 70 g of a methylhydrogenorganopolysiloxane represented by the following average structural formula:

(CH₃)_{1.82}(H)_{0.228}SiO_{0.972}

0.013 g of an ethanol solution (0.5%) of chloroplatinic acid divinyldisiloxane complex, and 61 g of an organic compound with a terminal unsaturated bond represented by the formula below:

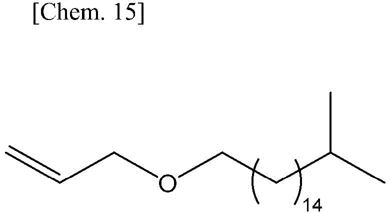

The mixture was aged at 80°C for 1 hour while performing stirring. Subsequently, 21 g of a glycerin compound with a terminal unsaturated bond represented by the formula below and 65 g of 2-propanol were added.

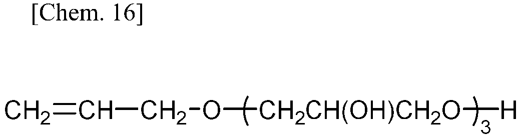

The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The organopolysiloxane obtained was dissolved into light liquid paraffin to give a 0.01% solution. The interfacial tension with water was measured 8 hours later by the Wilhelmy method, giving a value of 17.3 mN/m.

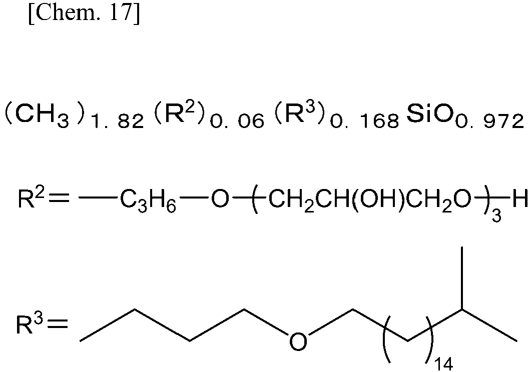

### [Production Example 2]

A reactor was charged with 100 g of a methylhydrogenorganopolysiloxane represented by the following average structural formula:

(CH₃)_{1.53}(H)_{0.59}SiO_{0.94}

0.02 g of an ethanol solution (0.5%) of chloroplatinic acid divinyldisiloxane complex, and 113 g of an organic compound with a terminal unsaturated bond represented by the formula below:

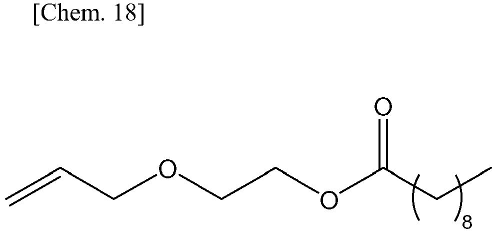

The mixture was aged at 80°C for 1 hour while performing stirring. Subsequently, 134 g of a glycerin compound with a terminal unsaturated bond represented by the formula below and 200 g of 2-propanol were added.

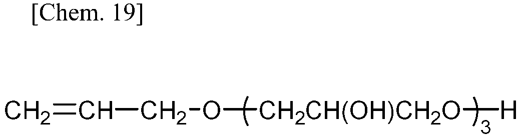

The mixture was aged at 80°C for 5 hours. After the disappearance of hydrosilyl groups was confirmed by ¹H-NMR, the mixture was stripped under reduced pressure to remove the solvent. Thus, a highly viscous liquid organopolysiloxane with the average structure below was obtained. The organopolysiloxane obtained was dissolved into light liquid paraffin to give a 0.01% solution. The interfacial tension with water was measured 8 hours later by the Wilhelmy method, giving a value of 8.9 mN/m.

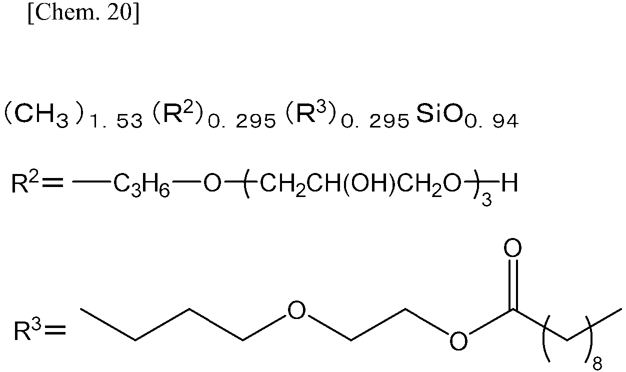

### [Production Example 3]

A reactor was charged with 250 g of a methylhydrogenorganopolysiloxane represented by the following average structural formula:

(CH₃)_{1.89}(H)_{0.11}SiO_{0.998}

0.1 g of an ethanol solution (0.5%) of chloroplatinic acid divinyldisiloxane complex, and 104 g of an organic compound with a terminal unsaturated bond represented by the formula below:

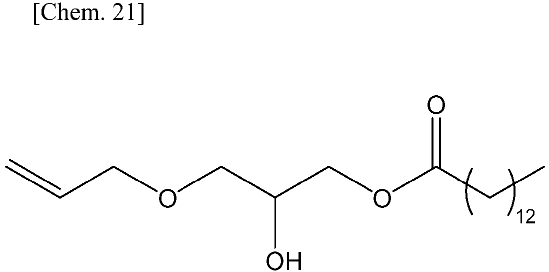

The mixture was aged at 80°C for 1 hour while performing stirring. Subsequently, 34.2 g of a glycerin compound with a terminal unsaturated bond represented by the formula below and 150 g of 2-propanol were added.

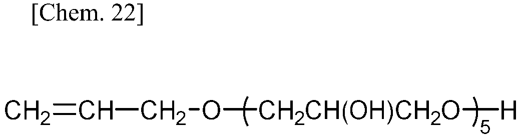

The mixture was aged at 80°C for 5 hours. After the disappearance of hydrosilyl groups was confirmed by ¹H-NMR, the mixture was stripped under reduced pressure to remove the solvent. Thus, a highly viscous liquid organopolysiloxane represented by the average structural formula below was obtained. The organopolysiloxane obtained was dissolved into light liquid paraffin to give a 0.01% solution. The interfacial tension with water was measured 8 hours later by the Wilhelmy method, giving a value of 23.4 mN/m.

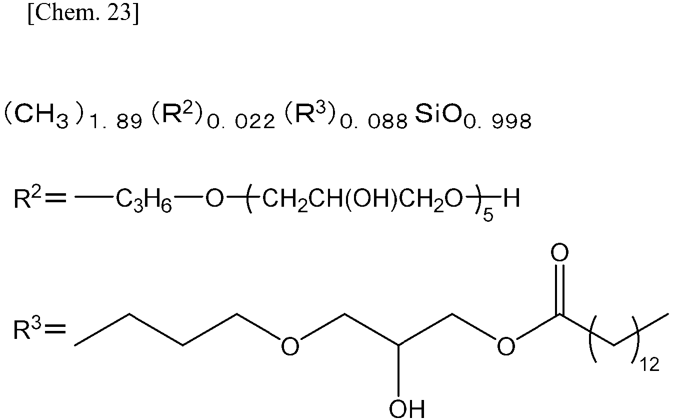

### [Production Example 4]

A reactor was charged with 300 g of a methylhydrogenorganopolysiloxane represented by the following average structural formula:

(CH₃)_{1.72}(H)_{0.31}SiO_{0.983}

0.1 g of an ethanol solution (0.5%) of chloroplatinic acid divinyldisiloxane complex, and 310 g of an organic compound with a terminal unsaturated bond represented by the formula below:

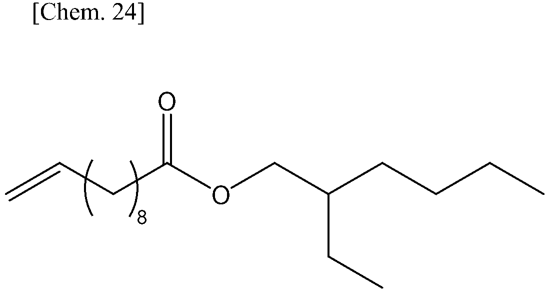

The mixture was aged at 80°C for 1 hour while performing stirring. Subsequently, 54 g of a glycerin compound with a terminal unsaturated bond represented by the formula below: 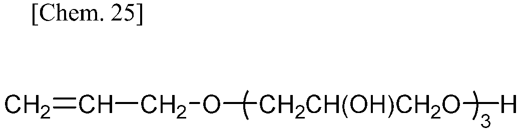 an amount of a siloxane compound with a terminal unsaturated bond represented by the formula below: 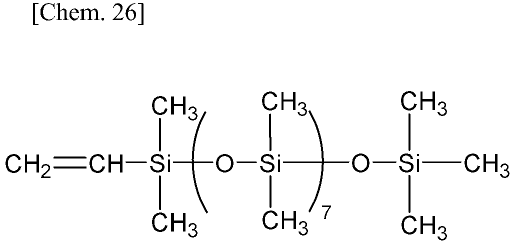 and 200 g of 2-propanol were added. The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The organopolysiloxane obtained was dissolved into light liquid paraffin to give a 0.01% solution. The interfacial tension with water was measured 8 hours later by the Wilhelmy method, giving a value of 16.4 mN/m.

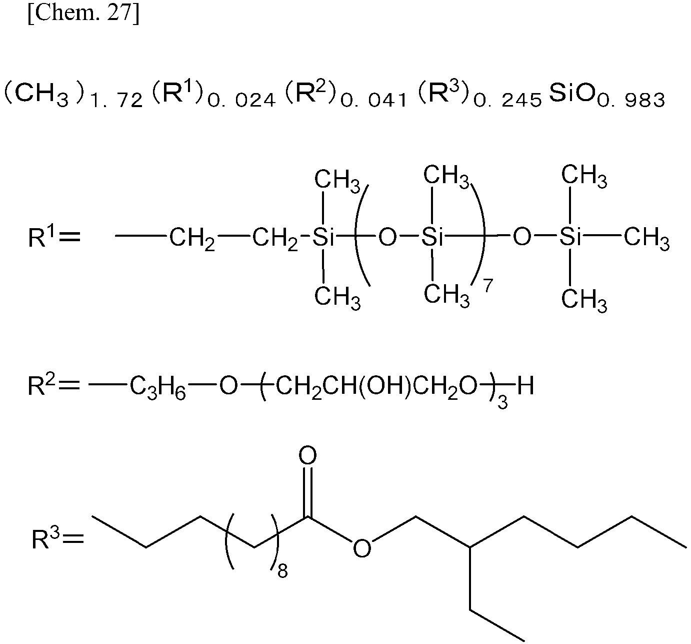

### [Production Example 5]

A reactor was charged with 150 g of a methylhydrogenorganopolysiloxane represented by the following average structural formula:

(CH₃)_{1.88}(H)_{0.209}SiO_{0.953}

0.1 g of an ethanol solution (0.5%) of chloroplatinic acid divinyldisiloxane complex, and 62 g of an organic compound with a terminal unsaturated bond represented by the formula below:

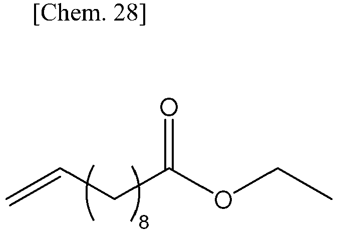

The mixture was aged at 80°C for 1 hour while performing stirring. Subsequently, 35.0 g of a glycerin compound with a terminal unsaturated bond represented by the formula below and 100 g of 2-propanol were added.

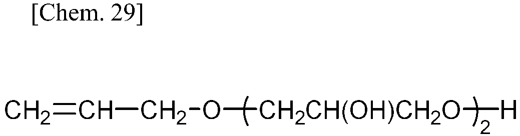

The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The organopolysiloxane obtained was dissolved into light liquid paraffin to give a 0.01% solution. The interfacial tension with water was measured 8 hours later by the Wilhelmy method, giving a value of 19.2 mN/m.

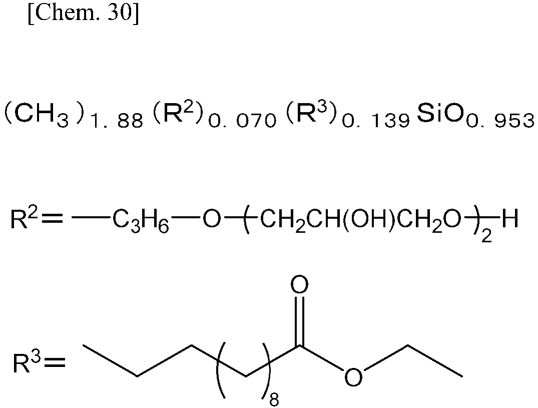

### [Comparative Production Example 1]

A reactor was charged with 70 g of a methylhydrogenorganopolysiloxane represented by the following average structural formula:

(CH₃)_{1.82}(H)_{0.228}SiO_{0.972}

0.013 g of an ethanol solution (0.5%) of chloroplatinic acid divinyldisiloxane complex, and 43 g of 1-hexadecene. The mixture was aged at 80°C for 1 hour while performing stirring. Subsequently, 21 g of a glycerin compound with a terminal unsaturated bond represented by the formula below and 65 g of 2-propanol were added.

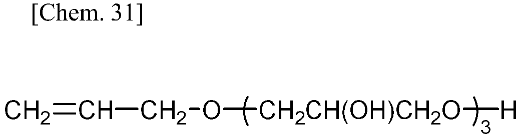

The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The organopolysiloxane obtained was dissolved into light liquid paraffin to give a 0.01% solution. The interfacial tension with water was measured 8 hours later by the Wilhelmy method, giving a value of 25.4 mN/m.

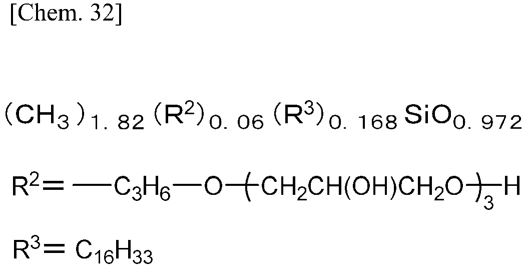

The advantageous effects of the present invention will be demonstrated below by way of Examples and Comparative Examples of cosmetics.

### [Examples 1 and 2, and Comparative Examples 1 and 2]

Water-in-oil (W/O) cosmetics were prepared according to the formulations described in Table 1. The water-in-oil (W/O) cosmetics obtained were evaluated as follows.

### (Production method)

A: Ingredients (1) were mixed together.
B: Ingredients (2) were mixed together.
C: The mixture obtained in B was added to the mixture obtained in A and was emulsified to give a water-in-oil (W/O) cosmetic.

### [Oil phase properties]

The mixture (1) obtained in step A was visually observed at 25°C and was rated as "○" when the mixture was uniform and as "×" when the mixture was cloudy and nonuniform.

### [Emulsifiability]

When the mixture (2) was emulsified in step C, whether the entire amount of the mixture (2) could be emulsified properly was determined. The emulsifiability was rated as "○" when the entire amount was emulsified and as "×" when the emulsification failed and part of the mixture (2) was present on the bulk surface.

**[Table 1]**

| Ingredients | | Example | | Comparative Example | |
|---|---|---|---|---|---|
| | | 1 | 2 | 1 | 2 |
| (1) | Organopolysiloxane of Production Example 1 | 2.0 | | | |
| | Organopolysiloxane of Production Example 5 | | 2.0 | | |
| | Organopolysiloxane of Comparative Production Example 1 | | | 2.0 | |
| | KF-6105 (Note 1) | | | | 2.0 |
| | Ethylhexyl methoxycinnamate | 12.0 | 12.0 | 12.0 | 12.0 |
| | KF-56A (Note 2) | 6.0 | 6.0 | 6.0 | 6.0 |
| (2) | BG | 5.0 | 5.0 | 5.0 | 5.0 |
| | Sodium chloride | 0.5 | 0.5 | 0.5 | 0.5 |
| | Water | 74.5 | 74.5 | 74.5 | 74.5 |
| Total | | 100.0 | 100.0 | 100.0 | 100.0 |
| | Oil phase properties | ○ | ○ | × | × |
| | Emulsifiability | ○ | ○ | × | × |

| | | | | | |
|---|---|---|---|---|---|
| (Note 1) Shin-Etsu Chemical Co., Ltd.: Lauryl polyglyceryl-3 polydimethylsiloxyethyl dimethicone (Note 2) Shin-Etsu Chemical Co., Ltd.: Diphenylsiloxyphenyl trimethicone | | | | | |

As clear from Table 1, the cosmetics of Examples 1 and 2 had good oil phase properties and successfully formed emulsions in spite of high loading of organic ultraviolet absorber and a small amount of compatibilizer.

### [Examples 3 and 4, and Comparative Examples 3 and 4]

Water-in-oil (W/O) cosmetics were prepared according to the formulations described in Table 2. The water-in-oil (W/O) cosmetics obtained were evaluated as follows.

### (Production method)

A: Ingredients (1) were mixed together.
B: Ingredients (2) were mixed together.
C: The mixture obtained in B was added to the mixture obtained in A and was emulsified to give a water-in-oil (W/O) cosmetic.

### [Solubility]

The solubility of the oil phase obtained in step A was evaluated based on its transparency. The transparency is the total light transmittance measured with respect to a 1 cm thick cell filled with the sample in accordance with the method described in JIS K7361-1: 1997. The solubility was rated as "○" when the transmittance was 80% or more and as "×" when the transmittance was less than 80%.

### [Stability]

The cosmetics were each loaded into a 30 mL vial and stored in a thermostatic chamber at 50°C for two weeks, after which the viscosity was measured.

The stability was rated as "⊚" when the change in viscosity compared to immediately after the preparation was 0% or more and less than 15%, as "○" when the viscosity change was 15% or more and less than 20%, as "△" when the viscosity change was 20% or more and less than 30%, and as "×" when the cosmetic had been separated or the viscosity change was 30% or more. Characteristic evaluations were cancelled for the formulations with the stability rating ×.

The viscosities immediately after preparation of the viscous products in Examples and Comparative Examples were in the range of 10,000 to 200,000 mPa·s.

The viscosity for this evaluation was measured using a B-type viscometer (TVB-10, manufactured by Toki Sangyo Co., Ltd.) with a spindle No. 4, at 6 rpm and 25°C for a measurement time of 60 seconds.

### [Feeling on use]

The feeling on use (spreadability) of the cosmetics was evaluated by a panel of ten experts based on the scoring criteria below. The scores of the ten experts were averaged and the results were evaluated according to the evaluation criteria below. The results are described in the table.

### [Scoring criteria]

5 Points: Very good
4 Points: Good
3 Points: Fair
2 Points: Rather poor
1 Point: Poor

The average score was evaluated based on the following ○ × criteria.

### [Evaluation criteria]

⊚: The average score is 4.5 points or more.
○: The average score is 3.5 points or more and less than 4.5 points.
△: The average score is 2.5 points or more and less than 3.5 points.
×: The average score is 1.5 points or more and less than 2.5 points.
××: The average score is less than 1.5 points.
"△" and higher ratings were accepted.

**[Table 2]**

| Ingredients | | Example | | Comparative Example | |
|---|---|---|---|---|---|
| | | 3 | 4 | 3 | 4 |
| (1) | Organopolysiloxane of Production Example 1 | 2.0 | | | |
| | Organopolysiloxane of Production Example 5 | | 2.0 | | |
| | Organopolysiloxane of Comparative Production Example 1 | | | 2.0 | |
| | KF-6105 (Note 1) | | | | 2.0 |
| | Jojoba seed oil | 23.0 | 23.0 | 23.0 | 23.0 |
| (2) | BG | 5 | 5 | 5 | 5 |
| | Sodium chloride | 0.5 | 0.5 | 0.5 | 0.5 |
| | Water | 69.5 | 69.5 | 69.5 | 69.5 |
| Total | | 100.0 | 100.0 | 100.0 | 100.0 |
| Characteristic evaluations | Solubility | ○ | ○ | × | × |
| | Stability | ⊚ | ○ | × | × |
| | Feeling on use | ○ | ⊚ | - | - |

As clear from Table 2, the cosmetics of Examples 3 and 4 had good oil phase properties, and the emulsions were stable in spite of high loading of natural oil and also had excellent feeling on use.

### [Example 5] Sunscreen

| | Composition | % |
|---|---|---|
| 1. | Dimethylpolysiloxane (2 cs) | 10 |
| 2. | Ethylhexyl triazone | 3 |
| 3. | Diethylamino hydroxybenzoyl hexyl benzoate | 2 |
| 4. | Octocrylene | 2.5 |
| 5. | Ethylhexyl salicylate | 5.5 |
| 6. | KSG-710 (Note 1) | 3.5 |
| 7. | KSG-18A (Note 2) | 3 |
| 8. | Organopolysiloxane of Production Example 1 | 1.5 |
| 9. | BG | 3 |
| 10. | Sodium citrate | 0.2 |
| 11. | Sodium chloride | 0.5 |
| 12. | Ethanol | 5 |
| 13. | Water | 56 |
| | Total | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer | | |

### (Production method)

A: Ingredients 1 to 8 were mixed uniformly.
B: Ingredients 9 to 12 were added to ingredient 13 and were dissolved.
C: The mixture obtained in B was gradually added to the mixture obtained in A and emulsified to give a sunscreen.

The sunscreen obtained as described above spread lightly, left the skin dry without stickiness, and was free from changes due to temperature or aging, thus being shown to have excellent usability and stability.

### [Example 6] Stick-type W/O foundation

| Composition | | % |
|---|---|---|
| 1. | KSG-710 (Note 1) | 5 |
| 2. | KSG-18A (Note 2) | 3 |
| 3. | Organopolysiloxane of Production Example 1 | 2 |
| 4. | Cyclopentasiloxane | 10 |
| 5. | Inulin stearate | 3 |
| 6. | Synthetic wax | 7 |
| 7. | t-Butyl methoxydibenzoylmethane | 2 |
| 8. | Ethylhexyl salicylate | 4 |
| 9. | Bisethylhexyloxyphenol methoxyphenyl triazine | 2 |
| 10. | Polysilicone-15 | 2 |
| 11. | Homosalate | 5 |
| 12. | Isotridecyl isononanoate | 2 |
| 13. | KP-578 (Note 3) | 0.3 |
| 14. | KTP-09R (Note 4) | 0.3 |
| 15. | KTP-09Y (Note 4) | 0.6 |
| 16. | KTP-09B (Note 4) | 0.1 |
| 17. | KTP-09W (Note 4) | 7 |
| 18. | BG | 7 |
| 19. | Glycerin | 5 |
| 20. | Sodium citrate | 0.2 |
| 21. | Sodium chloride | 1 |
| 22. | Disodium edetate | 0.1 |
| 23. | Water | Balance |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: (Acrylates/ethylhexyl acrylate/dimethicone methacrylate) copolymer (Note 4) Shin-Etsu Chemical Co., Ltd.: KF-9909-treated coloring inorganic pigments, W: white, R: red, Y: yellow, B: black | | |

### (Production method)

A: Ingredients 12 to 17 were dispersed using a three-roll mill.
B: Ingredients 1 to 11 were dissolved at 90°C, and the mixture obtained in A was added. The resultant mixture was mixed uniformly.
C: Ingredients 18 to 23 were mixed uniformly.
D: The mixture obtained in C was added to the mixture obtained in B and emulsified. The emulsion was loaded into a stick-shaped container. A W/O stick foundation was thus obtained.

The W/O stick foundation obtained had good feeling on use and good applicability and was excellent in storage stability.

### [Example 7] Sunscreen lotion (shaking)

| Composition | | % |
|---|---|---|
| 1. | Dimethylpolysiloxane (1.5 cs) | 29.5 |
| 2. | Dimethylpolysiloxane (6 cs) | 2 |
| 3. | KSG-18A (Note 1) | 3 |
| 4. | Organopolysiloxane of Production Example 5 | 2 |
| 5. | Ethylhexyl methoxycinnamate | 7.5 |
| 6. | Diethylamino hydroxybenzoyl hexyl benzoate | 1 |
| 7. | Octocrylene | 2.5 |
| 8. | Disteardimonium hectorite | 1 |
| 9. | KP-545 (Note 2) | 2 |
| 10. | KSP-105 (Note 3) | 0.5 |
| 11. | SPD-T5 (Note 4) | 5 |
| 12. | SPD-Z5 (Note 5) | 10 |
| 13. | BG | 3 |
| 14. | Sodium citrate | 0.2 |
| 15. | Sodium chloride | 0.5 |
| 16. | Ethanol | 5 |
| 17. | Water | 25.3 |
| Total | | 100.0 |

| | | |
|---|---|---|
| (Note 1) Shin-Etsu Chemical Co., Ltd.: Mixture of 80-90% diphenylsiloxyphenyl trimethicone + 10-20% (dimethicone/phenyl vinyl dimethicone) crosspolymer (Note 2) Shin-Etsu Chemical Co., Ltd.: Solution of 70% cyclopentasiloxane + 30% (acrylates/dimethicone) copolymer (Note 3) Shin-Etsu Chemical Co., Ltd.: (Vinyl dimethicone/methicone silsesquioxane) crosspolymer (Note 4) Shin-Etsu Chemical Co., Ltd.: Dispersion of 40% titanium oxide fine particles in cyclopentasiloxane (Note 5) Shin-Etsu Chemical Co., Ltd.: Dispersion of 60% zinc oxide fine particles in cyclopentasiloxane | | |

### (Production method)

A: Ingredients 1 to 9 were mixed uniformly.
B: Ingredient 10 was added to the mixture obtained in A and dispersed uniformly.
C: Ingredients 13 to 16 were added to ingredient 17 and were dissolved.
D: The mixture obtained in C was gradually added to the mixture obtained in B to form an emulsion, to which ingredients 11 and 12 were added. A sunscreen lotion was thus obtained.

The sunscreen lotion obtained as described above spread lightly, left the skin dry without stickiness, and was free from changes due to temperature or aging, thus being shown to have excellent usability and stability.

### [Example 8] Sunscreen cream

| Composition | | % |
|---|---|---|
| 1. | Dimethylpolysiloxane (2 cs) | 30 |
| 2. | Squalane | 3 |
| 3. | Organopolysiloxane of Production Example 2 | 4 |
| 4. | Ethylhexyl methoxycinnamate | 7.5 |
| 5. | t-Butyl methoxydibenzoylmethane | 3 |
| 6. | Polysilicone-15 | 1 |
| 7. | Distearyldimonium chloride | 1 |
| 8. | Tocophenol acetate | 0.1 |
| 9. | Ethanol | 1 |
| 10. | Sodium citrate | 0.5 |
| 11. | Magnesium sulfate | 0.5 |
| 12. | Preservative | 0.3 |
| 13. | Water | 48.1 |
| Total | | 100.0 |

### (Production method)

A: Ingredients 1 to 8 were mixed uniformly.
B: Ingredients 9 to 12 were dissolved uniformly into ingredient 13.
C: While performing stirring, the mixture obtained in B was gradually added to the mixture obtained in A and emulsified to give a sunscreen cream.

The sunscreen cream obtained as described above had a fine texture, spread lightly, gave a moist and dewy feel, and was free from stickiness and caught no sand, thus being shown to have very good usability. Furthermore, the sunscreen cream was free from changes due to temperature or aging, thus being shown to have excellent stability.

### [Example 9] Sunscreen cream

| Composition | | % |
|---|---|---|
| 1. | Decamethylcyclopentasiloxane | 16 |
| 2. | KSG-310 (Note 1) | 3.5 |
| 3. | KSG-18A (Note 2) | 3 |
| 4. | Organopolysiloxane of Production Example 3 | 1.5 |
| 5. | KF-7312J (Note 3) | 3 |
| 6. | Ethylhexyl methoxycinnamate | 7.5 |
| 7. | t-Butyl methoxydibenzoylmethane | 3 |
| 8. | Ethylhexyl salicylate | 3 |
| 9. | BG | 5 |
| 10. | Sodium citrate | 0.5 |
| 11. | Sodium chloride | 1 |
| 12. | Preservative | 0.3 |
| 13. | Water | 52.7 |
| Total | | 100.0 |

| | | |
|---|---|---|
| (Note 1) Shin-Etsu Chemical Co., Ltd.: Mixture of 65-75% mineral oil + 25-35% (PEG-15/lauryl dimethicone) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: Solution of 50% trimethylsiloxysilicate in cyclopentasiloxane | | |

### (Production method)

A: Ingredients 1 to 8 were mixed uniformly.
B: Ingredients 9 to 12 were dissolved uniformly into ingredient 13.
C: While performing stirring, the mixture obtained in B was gradually added to the mixture obtained in A and emulsified to give a sunscreen cream.

The sunscreen cream obtained as described above spread lightly, gave a dewy feel without stickiness, and formed a uniform and highly water-repellent film, thus offering long-lasting cosmetic and ultraviolet protective effects. Furthermore, the sunscreen cream was free from changes due to temperature or aging. Thus, the sunscreen cream was shown to have excellent usability and stability.

### [Example 10] Eyeliner

| Composition | | % |
|---|---|---|
| 1. | Decamethylcyclopentasiloxane | 22 |
| 2. | Dimethylpolysiloxane (6 CS) | 5 |
| 3. | KSG-42A (Note 1) | 5 |
| 4. | Jojoba oil | 2 |
| 5. | Organopolysiloxane of Production Example 4 | 3 |
| 6. | KF-9901 (Note 2) treated black iron oxide | 20 |
| 7. | Ethanol | 5 |
| 8. | Preservative | 0.1 |
| 9. | Water | 37.9 |
| Total | | 100.0 |

| | | |
|---|---|---|
| (Note 1) Shin-Etsu Chemical Co., Ltd.: Mixture of 75-85% isododecane + 15-25% (vinyl dimethicone/lauryl dimethicone) crosspolymer (Note 2) Shin-Etsu Chemical Co., Ltd.: Hydrogen dimethicone | | |

### (Production method)

A: Ingredients 1 to 5 were mixed under heating conditions, and ingredient 6 was added and dispersed uniformly.
B: Ingredients 7 to 9 were dissolved under heating conditions.
C: While performing stirring, the mixture obtained in B was gradually added to the mixture obtained in A to form an emulsion. An eyeliner was thus obtained.

The eyeliner obtained as described above spread lightly without feeling oily or powdery, and gave a dewy and refreshing feeling on use. Furthermore, the eyeliner had good water resistance, water repellency, and perspiration resistance and thus had good durability and was resistant to coming off. Furthermore, the eyeliner was free from changes due to temperature or aging, thus being shown to have excellent stability.

### [Example 11] Lip cream

| Composition | | % |
|---|---|---|
| 1. | Dextrin (palmitate/ethylhexanoate) (Note 1) | 9 |
| 2. | Triethylhexanoin | 7 |
| 3. | KP-545 (Note 2) | 5 |
| 4. | KSG-43 (Note 3) | 8 |
| 5. | Organopolysiloxane of Production Example 1 | 2 |
| 6. | Decamethylcyclopentasiloxane | 46 |
| 7. | 1,3-Butylene glycol | 5 |
| 8. | Purified water | 10.8 |
| 9. | Colorant | 1.2 |
| 10. | Titanium oxide-coated mica | 6 |
| Total | | 100.0 |

| | | |
|---|---|---|
| (Note 1) Chiba Flour Milling Co., Ltd.: Rheopearl TT (Note 2) Shin-Etsu Chemical Co., Ltd.: Solution of 70% cyclopentasiloxane + 30% (acrylates/dimethicone) copolymer (Note 3) Shin-Etsu Chemical Co., Ltd.: Mixture of 65-75% triethylhexanoin + 25-35% (vinyl dimethicone/lauryl dimethicone) crosspolymer | | |

### (Production method)

A: Ingredient 9 was mixed with a portion of ingredient 2 and the mixture was dispersed with a roller mill. The dispersion obtained was mixed with ingredient 1, the remainder of ingredient 2, and ingredients 3 to 6 under heating conditions.
B: Ingredients 7 and 8 were heated and added to the mixture obtained in A to form an emulsion, which was then cooled.
C: Ingredient 10 was added to the emulsion obtained in B. A lip cream was thus obtained.

The lip cream obtained spread lightly, was free from stickiness or oily feel, and formed a long-lasting film on the lips.

### [Example 12] Lipstick

| Composition | | % |
|---|---|---|
| 1. | Dimethylpolysiloxane (2 cs) | 28.8 |
| 2. | Caprylyl methicone | 10 |
| 3. | Diisostearyl malate | 5 |
| 4. | Candelilla wax | 10 |
| 5. | Polyethylene | 7 |
| 6. | Microcrystalline wax | 2 |
| 7. | KF-7312J (Note 1) | 20 |
| 8. | KSG-43 (Note 2) | 5 |
| 9. | Organopolysiloxane of Production Example 5 | 3 |
| 10. | Mica | 8 |
| 11. | Colorant | 1.2 |
| Total | | 100.0 |

| | | |
|---|---|---|
| (Note 1) Shin-Etsu Chemical Co., Ltd.: Solution of 50% trimethylsiloxysilicate in cyclopentasiloxane (Note 2) Shin-Etsu Chemical Co., Ltd.: Mixture of 65-75% triethylhexanoin + 25-35% (vinyl dimethicone/lauryl dimethicone) crosspolymer | | |

### (Production method)

A: Ingredient 11 was mixed with a portion of ingredient 2 and the mixture was dispersed with a roll mill. The dispersion obtained was mixed with ingredient 1, the remainder of ingredient 2, and ingredients 3 to 10 under heating conditions.
B: The mixture was poured into a mold and was cooled to solidify. A lipstick was thus obtained.

The lipstick obtained spread well, formed a uniform film, and had high water repellency to give long-lasting cosmetic effects. The lipstick was also shown to have excellent storage stability.

### [Example 13] Water-breaking W/O foundation

| Composition | | % |
|---|---|---|
| 1. | KSG-710 (Note 1) | 4 |
| 2. | KSG-18A (Note 2) | 1 |
| 3. | Organopolysiloxane of Production Example 5 | 0.1 |
| 4. | Ethylhexyl methoxycinnamate | 7.5 |
| 5. | Dimethylpolysiloxane (2 cs) | 5.6 |
| 6. | Decamethylcyclopentasiloxane | 4.8 |
| 7. | KF-6115 (Note 3) | 0.3 |
| 8. | KTP-09W (Note 4) | 5.1 |
| 9. | KTP-09Y (Note 4) | 0.58 |
| 10. | KTP-09R (Note 4) | 0.25 |
| 11. | KTP-09B (Note 4) | 0.07 |
| 12. | Hydrophobized zinc oxide fine particles (Note 5) | 6 |
| 13. | BG | 8 |
| 14. | Sodium citrate | 0.2 |
| 15. | Magnesium sulfate | 0.5 |
| 16. | Water | 56 |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: Lauryl polyglyceryl-3 polydimethylsiloxyethyl dimethicone (Note 4) Shin-Etsu Chemical Co., Ltd.: KF-9909-treated coloring inorganic pigments, W: white, R: red, Y: yellow, B: black (Note 5) Shin-Etsu Chemical Co., Ltd.: AES-3083-treated | | |

### (Production method)

| | |
|---|---|
| Preparation of oil phase: | Ingredients 1 to 5 were mixed uniformly. |
| Preparation of aqueous phase: | Ingredients 13 to 16 were mixed uniformly. |
| Preparation of paste phase: | Ingredients 8 to 12 were mixed in ingredients 6 and 7, and the mixture was dispersed using a three-roll mill. |

The aqueous phase was gently added to the oil phase to effect a phase change. After emulsification, the paste was mixed. A water-breaking W/O foundation was thus obtained.

The water-breaking W/O foundation obtained was shown to have a good feeling on use, a good water-breaking feel, and good applicability.

### [Example 14] W/O eye cream

| Composition | | % |
|---|---|---|
| 1. | KSG-840 (Note 1) | 3 |
| 2. | KSG-44 (Note 2) | 6 |
| 3. | Organopolysiloxane of Production Example 1 | 1.5 |
| 4. | Jojoba seed oil | 6 |
| 5. | Meadowfoam oil | 12 |
| 6. | Shea butter | 1 |
| 7. | KSP-300 (Note 3) | 2 |
| 8. | Sodium hyaluronate | 0.1 |
| 9. | Erythritol | 5 |
| 10. | Methylgluceth-10 | 3 |
| 11. | Phenoxyethanol | 0.3 |
| 12. | Pentylene glycol | 3 |
| 13. | Sodium chloride | 0.5 |
| 14. | Water | Balance |
| Total | | 100.0 |

| | | |
|---|---|---|
| (Note 1) Shin-Etsu Chemical Co., Ltd.: Mixture of 65-75% squalane + 25-35% (lauryl dimethicone/polyglycerin-3) crosspolymer (Note 2) Shin-Etsu Chemical Co., Ltd.: Mixture of 65-75% squalane + 25-35% (vinyl dimethicone/lauryl dimethicone) crosspolymer (Note 3) Shin-Etsu Chemical Co., Ltd.: (Diphenyl dimethicone/vinyl diphenyl dimethicone/silsesquioxane) crosspolymer | | |

### (Production method)

A: Ingredients 1 to 7 were mixed uniformly.
B: Ingredients 8 to 14 were mixed uniformly.
C: The mixture obtained in B was added to the mixture obtained in A and emulsified to give a W/O eye cream.

The W/O eye cream obtained had a good feeling on use and good applicability, and was excellent in storage stability.

### [Example 15] W/O sunscreen milk

| Composition | | % |
|---|---|---|
| 1. | KSG-710 (Note 1) | 3 |
| 2. | KSG-18A (Note 2) | 2 |
| 3. | Organopolysiloxane of Production Example 5 | 1 |
| 4. | Sunflower seed oil | 2 |
| 5. | Dimethylpolysiloxane (6 cs) | 5 |
| 6. | KF-9021L (Note 3) | 2 |
| 7. | Isotridecyl isononanoate | 5 |
| 8. | SPD-T5L (Note 4) | 20 |
| 9. | SPD-Z5L (Note 5) | 28 |
| 10. | Dipropylene glycol | 2 |
| 11. | Sodium citrate | 0.2 |
| 12. | Sodium chloride | 1 |
| 13. | Arbutin | 2 |
| 14. | Dipotassium glycyrrhizinate | 0.2 |
| 15. | Sodium pyrosulfite | 0.05 |
| 16. | Purified water | Balance |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 85% diphenylsiloxyphenyl trimethicone + 15% (dimethicone/phenyl vinyl dimethicone) crosspolymer (Note 3) Shin-Etsu Chemical Co., Ltd.: Solution of 50% trimethylsiloxysilicate in dimethylpolysiloxane (2 cs) (Note 4) Shin-Etsu Chemical Co., Ltd.: Dispersion of 40% titanium oxide fine particles in dimethylpolysiloxane (2 cs) (Note 5) Shin-Etsu Chemical Co., Ltd.: Dispersion of 60% zinc oxide fine particles in dimethylpolysiloxane (2 cs) | | |

### (Production method)

A: Ingredients 1 to 7 were mixed uniformly.
B: Ingredients 10 to 16 were mixed uniformly.
C: The mixture obtained in B was added to the mixture obtained in A and emulsified. Ingredients 8 and 9 were added, and the resultant mixture was mixed uniformly to give a W/O sunscreen milk.

The W/O sunscreen milk obtained had a good feeling on use and good applicability, and was excellent in storage stability.

### [Example 16] W/O liquid foundation

| Composition | | % |
|---|---|---|
| 1. | KSG-270 (Note 1) | 3.5 |
| 2. | KSG-18A (Note 2) | 5 |
| 3. | Organopolysiloxane of Production Example 1 | 2.5 |
| 4. | Organically modified clay mineral | 1 |
| 5. | Dimethylpolysiloxane (2 cs) | 21 |
| 6. | Octocrylene | 5 |
| 7. | Polysilicone-15 | 2 |
| 8. | KSP-101 (Note 3) | 2 |
| 9. | Isononyl isononanoate | 3.7 |
| 10. | KP-578 (Note 4) | 0.3 |
| 11. | KTP-09W (Note 5) | 14 |
| 12. | KTP-09R (Note 5) | 0.2 |
| 13. | KTP-09Y (Note 5) | 0.5 |
| 14. | KTP-09B (Note 5) | 1 |
| 15. | BG | 5 |
| 16. | Sodium citrate | 0.2 |
| 17. | Sodium chloride | 1 |
| 18. | Water | Balance |
| Total | | 100.0 |

| | | |
|---|---|---|
| (Note 1) Shin-Etsu Chemical Co., Ltd.: Mixture of 15-25% (dimethicone/(PEG-10/15)) crosspolymer and 75-85% diphenylsiloxyphenyl trimethicone (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: (Vinyl dimethicone/methicone silsesquioxane) crosspolymer (Note 4) Shin-Etsu Chemical Co., Ltd.: (Acrylates/ethylhexyl acrylate/dimethicone methacrylate) copolymer (Note 5) Shin-Etsu Chemical Co., Ltd.: KF-9909-treated coloring inorganic pigments, W: white, R: red, Y: yellow, B: black | | |

### (Production method)

A: Ingredients 9 to 14 were dispersed with a three-roll mill.
B: Ingredients 1 to 8 were mixed uniformly.
C: Ingredients 15 to 18 were mixed uniformly.
D: The mixture obtained in C was added to the mixture obtained in B and emulsified, and the mixture obtained in A was added. A W/O liquid foundation was thus obtained.

The W/O liquid foundation obtained had a good feeling on use and good applicability, and was excellent in storage stability.

### [Example 17] W/O solid foundation

| Composition | | % |
|---|---|---|
| 1. | KSG-710 (Note 1) | 5 |
| 2. | KSG-19 (Note 2) | 3 |
| 3. | Organopolysiloxane of Production Example 1 | 2 |
| 4. | SPD-T7 (Note 3) | 10 |
| 5. | KF-56A (Note 4) | 7.35 |
| 6. | Ceresin | 5 |
| 7. | Octyldodecyl myristate | 2 |
| 8. | KTP-09W (Note 5) | 7 |
| 9. | KTP-09B (Note 5) | 0.1 |
| 10. | KTP-09R (Note 5) | 0.3 |
| 11. | KTP-09Y (Note 5) | 0.6 |
| 12. | KP-545 (Note 6) | 0.3 |
| 13. | BG | 7 |
| 14. | Glycerin | 5 |
| 15. | Sodium citrate | 0.2 |
| 16. | Sodium chloride | 1 |
| 17. | Water | Balance |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% dimethicone (6 cs) + 10-20% (dimethicone/vinyl dimethicone) crosspolymer (Note 3) Shin-Etsu Chemical Co., Ltd.: Dispersion of 45% titanium oxide fine particles in cyclopentasiloxane (Note 4) Shin-Etsu Chemical Co., Ltd.: Diphenylsiloxyphenyl trimethicone (Note 5) Shin-Etsu Chemical Co., Ltd.: KF-9909-treated coloring inorganic pigments, W: white, R: red, Y: yellow, B: black (Note 6) Shin-Etsu Chemical Co., Ltd.: (Acrylates/dimethicone) copolymer | | |

### (Production method)

A: Ingredients 7 to 12 were dispersed with a three-roll mill.
B: Ingredients 1 to 6 were dissolved at 90°C, and the dispersion obtained in A was added. The resultant mixture was mixed uniformly.
C: Ingredients 13 to 17 were mixed uniformly.
D: The mixture obtained in C was added to the mixture obtained in B and emulsified to give a W/O solid foundation.

The W/O solid foundation obtained had a good feeling on use and good applicability, and was excellent in storage stability.

### [Example 18] Lipstick

| Composition | | % |
|---|---|---|
| 1. | Candelilla wax | 4 |
| 2. | Polyethylene | 2 |
| 3. | Microcrystalline wax | 3 |
| 4. | Ceresin | 7 |
| 5. | KP-561P (Note 1) | 15 |
| 6. | Organopolysiloxane of Production Example 4 | 3 |
| 7. | Macadamia nut oil | 28 |
| 8. | Diisostearyl malate | 10 |
| 9. | Hydrogenated polyisobutene | 10 |
| 10. | Isotridecyl isononanoate | 18 |
| 11. | Pearl pigment | 5 |
| 12. | Mica | 0.3 |
| 13. | Red 202 | 0.1 |
| 14. | Yellow 4 | 0.3 |
| 15. | KP-574 (Note 2) treated titanium oxide | 0.8 |
| 16. | KP-574 (Note 2) treated black iron oxide | 0.06 |
| 17. | Red 201 | 0.04 |
| 18. | KP-574 (Note 2) treated red iron oxide | 0.2 |
| 19. | Polyglvceryl-2 triisostearate | 1.2 |
| Total | | 100.0 |

| | | |
|---|---|---|
| (Note 1) Shin-Etsu Chemical Co., Ltd.: (Acrylates/stearyl acrylate/dimethicone methacrylate) copolymer (Note 2) Shin-Etsu Chemical Co., Ltd.: (Acrylates/tridecyl acrylate/triethoxysilylpropyl methacrylate/dimethicone methacrylate) copolymer | | |

### (Production method)

A: Ingredients 12 to 19 were dispersed using a roll.
B: Ingredients 1 to 10 were mixed uniformly at 95°C.
C: The mixture obtained in A and ingredient 11 were added to the mixture obtained in B. The resultant mixture was cooled to give a lipstick.

The lipstick obtained had a good feeling on use and good applicability, and was excellent in storage stability.

### [Example 19] W/O liquid foundation

| Composition | | % |
|---|---|---|
| 1. | KSG-710 (Note 1) | 3.5 |
| 2. | KSG-19 (Note 2) | 5 |
| 3. | Organopolysiloxane of Production Example 1 | 2.5 |
| 4. | Organically modified clay mineral | 0.8 |
| 5. | Dimethylpolysiloxane (2 cs) | 21 |
| 6. | Ethylhexyl palmitate | 7 |
| 7. | Glyceryl tri(caprylate/caprate) | 5 |
| 8. | KSP-101 (Note 3) | 2 |
| 9. | KF-56A (Note 4) | 7 |
| 10. | KF-6106 (Note 5) | 1 |
| 11. | KTP-09W (Note 6) | 10 |
| 12. | KTP-09R (Note 6) | 0.2 |
| 13. | KTP-09Y (Note 6) | 0.5 |
| 14. | KTP-09B (Note 6) | 0.1 |
| 15. | Metal soap-treated titanium oxide fine particles | 10 |
| 16. | BG | 5 |
| 17. | Sodium citrate | 0.2 |
| 18. | Sodium chloride | 1 |
| 19. | Water | Balance |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% dimethicone (6 cs) + 10-20% (dimethicone/vinyl dimethicone) crosspolymer (Note 3) Shin-Etsu Chemical Co., Ltd.: (Vinyl dimethicone/methicone silsesquioxane) crosspolymer (Note 4) Shin-Etsu Chemical Co., Ltd.: Diphenylsiloxyphenyl trimethicone (Note 5) Shin-Etsu Chemical Co., Ltd.: Lauryl polyglyceryl-3 polydimethylsiloxyethyl dimethicone (Note 6) Shin-Etsu Chemical Co., Ltd.: KF-9909-treated coloring inorganic pigments, W: white, R: red, Y: yellow, B: black | | |

### (Production method)

A: Ingredients 9 to 15 were dispersed with a three-roll mill.
B: Ingredients 1 to 8 were mixed uniformly.
C: Ingredients 16 to 19 were mixed uniformly.
D: The mixture obtained in C was added to the mixture obtained in B and emulsified, and the mixture obtained in A was added to give a W/O liquid foundation.

The W/O liquid foundation obtained had a good feeling on use and good applicability, and was excellent in storage stability.

### [Example 20] W/O cosmetic stick

| Composition | | % |
|---|---|---|
| 1. | KSG-830 (Note 1) | 5 |
| 2. | KSG-43 (Note 2) | 5 |
| 3. | Organopolysiloxane of Production Example 1 | 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | BG | 7 |
| 9. | PCA-Na | 2 |
| 10. | Diglycerin | 3 |
| 11. | Sodium citrate | 0.2 |
| 12. | Magnesium sulfate | 1 |
| 13. | Water | Balance |
| Total | | 100.0 |

| | | |
|---|---|---|
| (Note 1) Shin-Etsu Chemical Co., Ltd.: Mixture of 75-85% triethylhexanoin + 15-25% (lauryl dimethicone/polyglycerin-3) crosspolymer (Note 2) Shin-Etsu Chemical Co., Ltd.: Mixture of 65-75% triethylhexanoin + 25-35% (vinyl dimethicone/lauryl dimethicone) crosspolymer (Note 3) Shin-Etsu Chemical Co., Ltd.: Diphenylsiloxyphenyl trimethicone | | |

### (Production method)

A: Ingredients 1 to 7 were mixed uniformly at 90°C.
B: Ingredients 8 to 13 were mixed uniformly at 85°C.
C: The mixture obtained in B was added to the mixture obtained in A and emulsified. The emulsion was loaded into a stick container to give a W/O cosmetic stick.

The W/O cosmetic stick obtained had a good feeling on use and good applicability, and was excellent in storage stability.

### [Example 21] O/W sunscreen

| Composition | | % |
|---|---|---|
| 1. | Polysilicone-15 | 5 |
| 2. | Dicaprylyl carbonate | 5 |
| 3. | KF-56A (Note 1) | 5 |
| 4. | KF-7312K (Note 2) | 1 |
| 5. | Organopolysiloxane of Production Example 2 | 0.4 |
| 6. | Cocoalkyl (caprylate/caprate) | 5 |
| 7. | Stearic acid-treated titanium oxide fine particles | 4.5 |
| 8. | Polysorbate-60 | 2 |
| 9. | Sodium acrylate-sodium acryloyldimethyltaurate copolymer composition (Note 3) | 1 |
| 10. | 2% Solution of ammonium acryloyldimethyltaurate/VP copolymer | 15 |
| 11. | BG | 5 |
| 12. | Ethanol | 6 |
| 13. | Pentylene glycol | 1 |
| 14. | Ethylhexylglycerin | 0.15 |
| 15. | Purified water | Balance |
| Total | | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: Solution of 60% trimethylsiloxysilicate in dimethicone (6 cs) (Note 3) SEPPIC: SEPIGEL 305 | | |

### (Production method)

A: Ingredients 8 to 15 were mixed uniformly.
B: Ingredients 1 to 7 were dispersed uniformly.
C: The mixture obtained in B was added to the mixture obtained in A and emulsified to give an oil-in-water sunscreen.

The O/W sunscreen obtained had a good feeling on use and good applicability, and was excellent in storage stability.

### [Example 22] Hair oil

| Composition | | % |
|---|---|---|
| 1. | Organopolysiloxane of Production Example 3 | 3 |
| 2. | KF-56A (Note 1) | 7 |
| 3. | Diethylhexyl succinate | 10 |
| 4. | X-21-5613 (Note 2) | 1.5 |
| 5. | Tocopherol | 0.1 |
| 6. | Fragrance | 0.1 |
| 7. | Light liquid isoparaffin | Balance |
| Total | | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: Solution with high degree of polymerization dimethiconol | | |

### (Production method)

A: Ingredients 1 to 7 were mixed uniformly to give a hair oil.

The hair oil obtained had a good feeling on use and good applicability, and was excellent in storage stability.

### [Example 23] Lip color

| Composition | | % |
|---|---|---|
| 1. | KF-56A (Note 1) | Balance |
| 2. | Organopolysiloxane of Production Example 1 | 1 |
| 3. | KSP-100 (Note 2) | 4 |
| 4. | KMP-590 (Note 3) | 6 |
| 5. | Candelilla wax | 3 |
| 6. | Synthetic wax | 5 |
| 7. | Dipentaerythrityl hexa(hydroxystearate/stearate/rosinate) | 3 |
| 8. | Hydrogenated polyisobutene (18-mer) | 4 |
| 9. | Pentaerythrityl tetraisostearate | 10 |
| 10. | Isotridecyl isononanoate | 11 |
| 11. | Sericite | 1.4 |
| 12. | Polyglyceryl-2 triisostearate | 3 |
| 13. | Red No. 201 | 0.2 |
| 14. | Red No. 202 | 0.3 |
| 15. | Yellow No. 4 | 1.1 |
| Total | | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: (Vinyl dimethicone/methicone silsesquioxane) crosspolymer (Note 3) Shin-Etsu Chemical Co., Ltd.: Polymethylsilsesquioxane | | |

### (Production method)

A: Ingredients 11 to 15 were dispersed using a three-roll mill.
B: Ingredients 1 to 10 were mixed uniformly at 95°C.
C: The mixture obtained in A was added to the mixture obtained in B. The resultant mixture was mixed at 85°C, poured into a dish, and cooled to give a lip color.

The lip color obtained was confirmed to have good gloss, adhesion, and feeling on use, and also to be excellent in storage stability. The lip color can also be used as point makeup on areas other than the lips, for example, can be used as a cheek color by being applied thin.

### [Example 24] W/O hair cream

| Composition | | % |
|---|---|---|
| 1. | KSG-840 (Note 1) | 5 |
| 2. | KSG-44 (Note 2) | 1 |
| 3. | Organopolysiloxane of Production Example 4 | 0.5 |
| 4. | Olive fruit oil | 6 |
| 5. | Baobab seed oil | 1 |
| 6. | KF-4418 (Note 3) | 8 |
| 7. | KF-4422 (Note 4) | 6 |
| 8. | Lavender oil | 0.05 |
| 9. | Argania spinosa kernel oil | 0.05 |
| 10. | BG | 6 |
| 11. | Ethylhexylglycerin | 0.1 |
| 12. | Pentylene glycol | 3 |
| 13. | Magnesium sulfate | 0.5 |
| 14. | Sodium citrate | 0.2 |
| 15. | Water | Balance |
| Total | | 100.0 |

| | | |
|---|---|---|
| (Note 1) Shin-Etsu Chemical Co., Ltd.: Mixture of 65-75% squalane + 25-35% (lauryl dimethicone/polyglycerin-3) crosspolymer (Note 2) Shin-Etsu Chemical Co., Ltd.: Mixture of 65-75% squalane + 25-35% (vinyl dimethicone/lauryl dimethicone) crosspolymer (Note 3) Shin-Etsu Chemical Co., Ltd.: Caprylyl methicone (Note 4) Shin-Etsu Chemical Co., Ltd.: Ethyl methicone | | |

### (Production method)

A: Ingredients 1 to 9 were mixed uniformly.
B: Ingredients 10 to 15 were mixed uniformly.
C: The mixture obtained in B was added to the mixture obtained in A and emulsified to give a W/O hair cream.

The W/O hair cream obtained was dewy, provided easy combing, and had excellent storage stability.

## Claims

1. An organopolysiloxane represented by formula (1) below:
[Chem. 1]
R¹ₐR²_{b}R³_{c}SiO_{(4-a-b-c)/2} (1)
wherein R¹ independently at each occurrence is a group selected from C₁-C₁₀ alkyl groups, phenyl group, C₇-C₁₀ aralkyl groups, and formula (1-A) and formula (1-B) below: 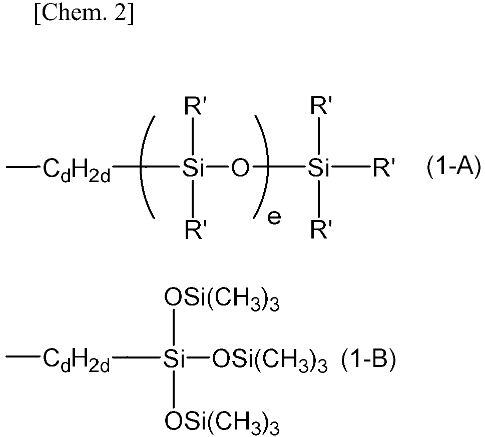 wherein each d is a number from 2 to 6, e is 1 to 100, and R' at each occurrence is a group selected from C₁-C₁₀ alkyl groups and phenyl group;
R² is a polyglycerol group represented by formula (2) below: 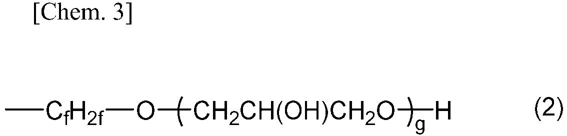 wherein f is a number from 3 to 12 and g is a number from 1 to 10;
R³ is an organic group represented by formula (3) below: 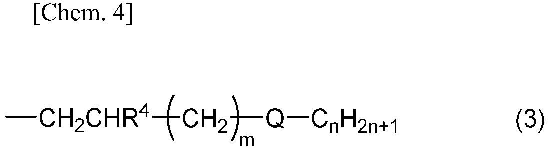 wherein R⁴ is a hydrogen atom or a methyl group,
Q is selected from formulas (3-1) to (3-4) below: 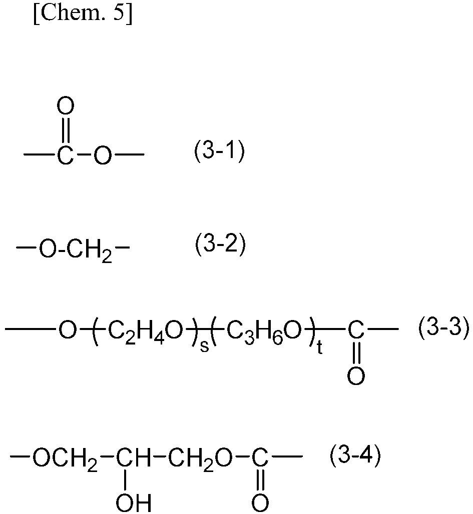
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22; and
a, b, and c are 1.0 ≤ a ≤ 2.5, 0.001 ≤ b ≤ 1.5, 0.001 ≤ c ≤ 1.5, and 1 < a + b + c < 4.

2. The organopolysiloxane according to claim 1, wherein the interfacial tension between a 0.01 wt% solution of the organopolysiloxane in a light liquid paraffin and water at 25°C, as measured by a Wilhelmy method 8 hours after the formation of interface, is 25 mN/m or less.

3. A cosmetic comprising the organopolysiloxane described in claim 1.

4. The cosmetic according to claim 3, further comprising an organic ultraviolet absorber.

5. The cosmetic according to claim 3 or 4, further comprising an oil ingredient that is liquid at 25°C.

6. The cosmetic according to claim 5, wherein the oil ingredient that is liquid at 25°C is an ingredient selected from hydrocarbon oils, natural oils, and esters.
